# EUROPEAN PATENT APPLICATION

(11) **EP 4 537 805 A1**
(43) Date of publication of application: **16.04.2025**
(21) Application number: 24202233.3
(22) Date of filing: 24.09.2024
(51) Int. Cl.: A61K 6/30, A61K 6/71, A61K 6/889

(54) **DENTAL RESIN-REINFORCED GLASS IONOMER CEMENT COMPOSITION FOR LUTING EXCELLENT IN ADHESIVE PROPERTY AND REMOVABILITY OF EXCESS CEMENT**

(30) Priority: 28.09.2023 JP 2023168927
(71) Applicant: Shofu Inc., Kyoto-shi, Kyoto 605-0983 (JP)
(72) Inventor: HIRATSUKA, Ken, Kyoto, 605-0983 (JP); KUMAGAI, Tomohiro, Kyoto, 605-0983 (JP); SAKAMOTO, Shuji, Kyoto, 605-0983 (JP); KIMOTO, Katsuya, Kyoto, 605-0983 (JP)
(74) Representative: Eisenführ Speiser

(57) **Abstract**

To provide a dental resin-reinforced glass ionomer cement composition for luting that can maintain a semi-set state, at which time excess cement can be removed, for a longer period of time, is less likely to tear even when the excess cement is thin, and also has excellent adhesive property to tooth substance, which is an important as basic property of the dental cement

To provide a dental resin-reinforced glass ionomer cement composition for luting consisting of a first agent including (a) aryl borate compound and (b) acid-reactive glass powder and a second agent including (c) polyalkenoic acid and (d) water, wherein at least one of the first agent and the second agent contains (e) polymerizable monomer, the content of the (c) polyalkenoic acid contained in the second agent based on the whole of the second agent is more than 20 % by mass and less than 70 % by mass, and the content of the (c) polyalkenoic acid contained in the second agent in the total of 100 parts by mass of the (c) polyalkenoic acid contained in the second agent and the (d) water contained in the second agent is more than 45 parts by mass and less than 75 parts by mass.

## Description

### BACKGROUND OF THE INVENTION

### Field of the Invention

The present invention relates to a dental resin-reinforced glass ionomer cement composition for luting that is useful as a dental cement for bonding and/or luting a dental prosthesis device to a tooth.

### Description of the Related Art

In a dental practice, cement materials have been used as a material which adheres and/or bonds a dental prosthesis device such as a crown, an inlay and a bridge to a tooth in which a form was partially lost by caries or breakages. Examples of the dental cements which are mainly used currently include an adhesive resin cement, a glass ionomer cement for luting and a resin-reinforced glass ionomer cement for luting. The most important basic property required for these dental cements is their adhesive property to tooth substance. Furthermore, the film thickness (the thickness of the cement layer between the tooth and the dental prosthesis device) is required to be thin so as to minimize lifting of the dental prosthesis device when applied to the tooth.

Among them, a resin-reinforced glass ionomer cement for luting contains the main components of an adhesive resin cement and a glass ionomer cement for luting, and is a useful material that has both the high mechanical characteristic of the former and the property of the latter, such as tooth substance reinforcement through the sustained release of fluoride. For example, Japanese Unexamined Patent Application Publication No. 2014-181190 discloses that a powder-liquid type dental resin-reinforced glass ionomer cement consisting of a powder material including fluoroaluminosilicate glass particles and polyalkenoic acid powder in a specific ratio and a liquid material including a polymerizable monomer having an acidic group, a polymerizable monomer not having an acidic group, a specific basic compound and water and exhibiting pH in a specific range, and containing a chemical polymerization initiator in the powder material and/or the liquid material is excellent in adhesive property to tooth substance and storage stability, and is useful for filling restorations and luting of prosthetic appliances.

The operation of attaching a dental prosthesis device to a tooth using this dental resin-reinforced glass ionomer cement for luting is performed by applying the cement kneaded material to the adhesive surface of the dental prosthesis device and then pressing the dental prosthesis device to the tooth. In this case, the kneaded material is applied in an excess amount larger than necessary amount so as to spread over the entire adhesive interface between the dental prosthesis device and the tooth. Therefore, when the dental prosthesis device is pressed against the tooth, an excess of the kneaded material protrudes from the joint portion between the dental prosthesis device and the tooth. This protruding excess kneaded material is called excess cement, and when it has set to a certain extent and is in a semi-set state, it is removed with a sharp-tipped instrument called a dental probe.

In addition, when the dental prosthesis device is a crown or a bridge, the joint portion between the dental prosthesis device and the tooth (hereinafter also referred to as the margin portion) is near or below the gingival margin on the tooth surface. Therefore, great care must be taken not to damage the surrounding gingiva with a dental probe when removing excess cement.

Removal of excess cement is even more difficult, especially on an approximal surface between teeth. Therefore, in addition to the above described basic properties (adhesive property to tooth substance, thin film thickness), a dental resin-reinforced glass ionomer cement is also required to be able to easily remove excess cement.

When the excess cement is not sufficiently removed, there is concern that the risk of periodontitis may increase (Thomas G. Wilson Jr., J Periodontol 2009, 80(9), 1388-1392). Thus, the removability of excess cement is an extremely important property since it significantly affects prognosis.

### SUMMARY OF THE INVENTION

### Technical Problem

As described above, the excess cement is removed when the excess cement reaches a semi-set state. This is because excess cement can be easily removed without the need for strong force, minimizing the risk of missed removal and gingival damage. Therefore, the longer a semi-set state during the set process of the excess cement, the more favorable because this allows for removal work to be performed with more time to spare. Furthermore, when the excess cement in a semi-set state has tear-resistant property, it is possible to remove the excess cement in one lump, therefore it is more preferable since it greatly reduces the burden of the work. This tear-resistant property is particularly useful when removing excess cement from approximal surface, where visibility is poor and there is a high risk of leaving excess cement behind.

In the conventional resin-reinforced glass ionomer cement for luting, a semi-set state is maintained for a certain period of time during the set process, but in the clinical case where it takes a long time to remove excess cement, such as when removing excess cement from multiple teeth at once, the time maintaining the semi-set state is insufficient. Furthermore, when the thickness of the excess cement becomes too thin, it may tear in removing, therefore more tear-resistant property has been required.

Therefore, an object of the present invention is to provide a dental resin-reinforced glass ionomer cement composition for luting that can maintain a semi-set state, at which time excess cement can be removed, for a longer period of time, is less likely to tear even when the excess cement is thin, and also has excellent adhesive property to tooth substance, which is an important as basic property of the dental cement.

### Solution to Problem

In light of the above described problems, as a result of intensive studies to solve the above problems, the present inventors have found that the above described problem can be solved by providing a dental resin-reinforced glass ionomer cement composition for luting that consists of a first agent including acid-reactive glass powder and a second agent including polyalkenoic acid and water, wherein at least one of the first agent and the second agent contains a polymerizable monomer, the first agent includes an aryl borate compound as a polymerization initiator, and the content of polyalkenoic acid in the second agent and the ratio of polyalkenoic acid to water contained in the second agent are within specific ranges, thereby completing the present invention.

That is, the above problem can be solved by the following component composition.

That is, the present invention provides a dental resin-reinforced glass ionomer cement composition for luting consisting of a first agent including (a) aryl borate compound and (b) acid-reactive glass powder and a second agent including (c) polyalkenoic acid and (d) water, wherein at least one of the first agent and the second agent contains (e) polymerizable monomer, the content of the (c) polyalkenoic acid contained in the second agent based on the whole of the second agent is more than 20 % by mass and less than 70 % by mass, and the content of the (c) polyalkenoic acid contained in the second agent in the total of 100 parts by mass of the (c) polyalkenoic acid contained in the second agent and the (d) water contained in the second agent is more than 45 parts by mass and less than 75 parts by mass.

### Advantageous Effects of Invention

The dental resin-reinforced glass ionomer cement composition for luting of the present invention can exhibit excellent adhesive property to tooth substance, and can maintain a semi-set state suitable for removing excess cement for a long period during the set process and therefore it is possible to remove excess cement in ample time. Furthermore, because it has a property that is less likely to tear even when the excess cement is thin, it can be easily removed even from adjacent surfaces between teeth, where removal of excess cement is otherwise difficult. This reduces the risk of developing periodontitis due to excess cement being left behind.

### DETAILED DESCRIPTION OF THE PREFERRED EMBODIMENTS

In the present specification, "polyalkenoic acid" means a polymer containing an ethylenically unsaturated monomer unit having an acidic group.

In the present specification, the term "(meth)acrylate" inclusively refers to both acrylate and methacrylate, the term "(meth)acryloyl" inclusively refers to both acryloyl and methacryloyl, and the term "(meth)acrylamide" inclusively refers to both acrylamide and methacrylamide.

The dental resin-reinforced glass ionomer cement composition for luting of the present invention consists of a first agent including (a) aryl borate compound and (b) acid-reactive glass powder and a second agent including (c) polyalkenoic acid and (d) water, wherein at least one of the first agent and the second agent contains (e) polymerizable monomer, the content of the (c) polyalkenoic acid contained in the second agent based on the whole of the second agent is more than 20 % by mass and less than 70 % by mass, and the content of the (c) polyalkenoic acid contained in the second agent in the total of 100 parts by mass of the (c) polyalkenoic acid contained in the second agent and the (d) water contained in the second agent is more than 45 parts by mass and less than 75 parts by mass.

By this component composition, it is possible to exhibit excellent adhesive property to tooth substance and to maintain a semi-set state suitable for removing excess cement for a long period during set process and to easily remove excess cement from margin portion since the excess cement in semi-set state has tear-resistant property.

The present invention will be described in detail below. The (a) aryl borate compound that can be used in the dental resin-reinforced glass ionomer cement composition for luting of the present invention is an organic boron compound that is represented by the following general formula (1) and has three or four aryl groups in one molecule. 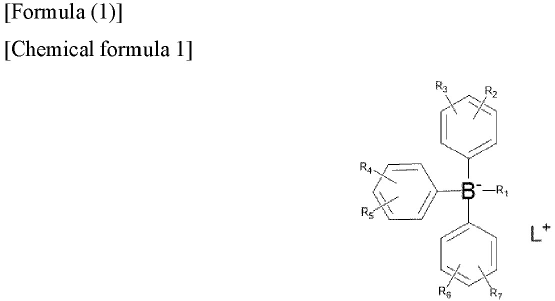 [In the formula, R₁ represents an alkyl group, an aryl group or an alkenyl group which may have a substituent, and R₂ to R₇ each independently represent a hydrogen atom, a halogen atom, a nitro group, or an alkyl group, phenyl group or alkoxy group which may have a substituent. L⁺ represents an alkali metal ion, a tertiary or quaternary ammonium ion, a quaternary pyridinium ion, a quaternary quinolinium ion or a quaternary phosphonium ion.]

Specific examples of the (a) aryl borate compound represented by the above formula (1) are given below.

Specific examples of borate compounds having three aryl groups in one molecule include sodium salts, lithium salts, potassium salts, tetrabutylammonium salts, tetramethylammonium salts, tetraethylammonium salts, tributylamine salts, triethanolamine salts, methylpyridinium salts, ethylpyridinium salts, butylpyridinium salts, methylquinolinium salts, ethylquinolinium salts and butylquinolinium salts of monoalkyl triphenylboron, monoalkyl tris (p-chlorophenyl) boron, monoalkyl tris (p-fluorophenyl) boron, monoalkyl tris [3,5-bis (trifluoromethyl) phenyl] boron, monoalkyl tris [3,5-bis (1,1,1,3,3,3-hexafluoro-2-methoxy-2-propyl) phenyl] boron, monoalkyl tris (p-nitrophenyl) boron, monoalkyl tris (m-nitrophenyl) boron, monoalkyl tris (p-butylphenyl) boron, monoalkyl tris (m-butylphenyl) boron, monoalkyl tris (p-butyloxyphenyl) boron, monoalkyl tris (m-butyloxyphenyl) boron, monoalkyl tris (p-octyloxyphenyl) boron and monoalkyl tris (m-octyloxyphenyl) boron (the alkyl group is an n-butyl group, an n-octyl group, an n-dodecyl group or the like), but are not limited thereto.

Specific examples of borate compounds having four aryl groups in one molecule include sodium salts, lithium salts, potassium salts, tetrabutylammonium salts, tetramethylammonium salts, tetraethylammonium salts, tributylamine salts, triethanolamine salts, methylpyridinium salts, ethylpyridinium salts, butylpyridinium salts, methylquinolinium salts, ethylquinolinium salts and butylquinolinium salts of tetraphenylboron, tetrakis (p-chlorophenyl) boron, tetrakis (p-fluorophenyl) boron, tetrakis [3,5-bis (trifluoromethyl) phenyl] boron, tetrakis [3,5-bis (1,1,1,3,3,3-hexafluoro-2-methoxy-2-propyl) phenyl] boron, tetrakis (p-nitrophenyl) boron, tetrakis (m-nitrophenyl) boron, tetrakis (p-butylphenyl) boron, tetrakis (m-butylphenyl) boron, tetrakis (p-butyloxyphenyl) boron, tetrakis (m-butyloxyphenyl) boron, tetrakis (p-octyloxyphenyl) boron and tetrakis (m-octyloxyphenyl)boron, but are not limited thereto.

These of the (a) aryl borate compound may be used alone or in a combination of a few kinds thereof. The dental resin-reinforced glass ionomer cement composition for luting of the present invention may not contain an aryl borate compound other than an organoboron compound having three or four aryl groups in one molecule.

It is preferable that a content of the (a) aryl borate compound is 0.01 % by mass or more and 30 % by mass or less and more preferably 0.1 % by mass or more and 25 % by mass or less based on the whole of the dental resin-reinforced glass ionomer cement composition for luting of the present invention. When the content of the (a) aryl borate compound is less than 0.01 % by mass, there is a case where the settability deteriorates and the mechanical characteristic decreases. Furthermore, when the content of the (a) aryl borate compound exceeds 30 % by mass, there is a case where the storage stability deteriorates.

The (b) acid-reactive glass powder that can be used in the dental resin-reinforced glass ionomer cement composition for luting of the present invention needs to contain an acid-reactive element such as a metal element, and a fluorine element. Because the (b) acid-reactive glass powder contains an acid reactive element, the acid-base reaction of the (b) acid-reactive glass powder with the acid group contained in the (c) polyalkenoic acid progresses in the presence of (d) water. Specific examples of the acid reactive element include sodium, potassium, calcium, strontium, barium, lanthanum, aluminum and zinc, but are not limited thereto. One or two or more kinds of these acid reactive elements may be contained and a content thereof is not particularly limitated.

Further, it is preferable that the (b) acid-reactive glass powder includes an X-ray impermeable element in order to impart X-ray contrast property to the dental resin-reinforced glass ionomer cement composition for luting of the present invention. Specific examples of the X-ray impermeable element include strontium, lanthanum, zirconium, titanium, yttrium, ytterbium, tantalum, tin, tellurium, tungsten and bismuth, but are not limited thereto. In addition, other element contained in the (b) acid-reactive glass powder is not particularly limited and the (b) acid-reactive glass powder in the present invention may include various elements.

Examples of the (b) acid-reactive glass powder include aluminosilicate glass, borosilicate glass, aluminoborate glass, boro aluminosilicate glass, phosphate glass, borate glass, silica glass which contain the above described acid reactive element, fluorine element and X-ray impermeable element, but are not limited thereto.

Further, a particle shape of the (b) acid-reactive glass powder is not particularly limited, but arbitral particle shapes such as spherical, needle-like, plate-like, ground-like, and scaly-shape may be used without any limitation. These (b) acid-reactive glass powder may be used alone or in a combination of a few kinds thereof.

A manufacturing method of the (b) acid-reactive glass powder is not particularly limited, and any of the (b) acid-reactive glass powder manufactured by any manufacturing methods such as a melting method, a vapor phase method and a sol-gel method may be used. Among them, the (b) acid-reactive glass powder manufactured by a melting method or a sol-gel method which can easily control a kind of element and the content thereof is preferably used.

The (b) acid-reactive glass powder may be ground to use in order to obtain a desirable particle diameter. A grinding method is not particularly limited, but an acid-reactive glass powder obtained by grinding which use any of wet or dry grinding methods may be used. Specifically, the particle diameter may be appropriately adjusted according to the desired property to be imparted to the dental resin-reinforced glass ionomer cement composition for luting of the present invention by grounding with a high speed rotating mill such as a hammer mill and a turbo-mill, a container driving type mill such as a ball mill, a planetary mill and a vibration mill, a medium stirring mill such as an attritor and a bead mill and a jet mill and the like.

In the dental resin-reinforced glass ionomer cement composition for luting of the present invention, it is preferable that the 50% particle diameter (D50) of the (b) acid-reactive glass powder is 0.1 µm or more and 30 µm or less, and more preferably 0.1 µm or more and 10 µm or less, in order to exhibit a thin film thickness. In the present specification, the term "50% particle diameter (D50)" means a particle diameter at which an integrated value from the small particle diameter side becomes 50% in a volume-based particle diameter distribution measured using a laser diffraction/scattering type particle size distribution measuring device and the like.

When the 50% particle diameter (D50) of the (b) acid-reactive glass powder is less than 0.1 µm, the surface area thereof increases and it becomes impossible to contain the acid-reactive glass powder in a large amount into the composition, therefore there is a case where the mechanical characteristic decreases and working time is shortened. Furthermore, when the 50% particle diameter (D50) of the (b) acid-reactive glass powder exceeds 30 µm, there is a case where the film thickness becomes thick and therefore the bonded and/or adhered dental prosthesis device is lifted to cause discomfort in the bite. The dental resin-reinforced glass ionomer cement composition for luting of the present invention may contain only acid-reactive glass powder having a 50% particle diameter (D50) of 0.1 µm or more and 30 µm or less as the (b) acid-reactive glass powder.

The (b) acid-reactive glass powder may be treated with various surface treatments, heat treatment, aggregating treatment in a liquid phase or a vapor phase and the like to such a range that the acid-base reaction of the (b) acid-reactive glass powder with the (c) polyalkenoic acid is not adversely affected, in order to adjust operability, a setting characteristic, a mechanical characteristic and the like of the dental resin-reinforced glass ionomer cement composition for luting of the present invention. These treatments can be performed alone or in a combination of a few kinds thereof, and the order in which the treatments are performed is not particularly limited. Among them, the surface treatment and the heat treatment are preferable because it is easy to control various characteristics and those are superior in productivity.

Specific examples of the surface treatment of the (b) acid-reactive glass powder include washing with an acid such as phosphoric acid or acetic acid, surface treatment with an acidic compound such as tartaric acid or polycarboxylic acid, surface treatment with a fluoride such as aluminum fluoride and surface treatment with a silane compound such as (meth) acryloyloxymethyl trimethoxysilane, 3-(meth) acryloyloxypropyl trimethoxysilane, 8-(meth) acryloyloxyoctyl trimethoxysilane, 3-mercaptopropyl trimethoxysilane, tetramethoxysilane, tetraethoxysilane, partially hydrolyzed oligomer of tetramethoxysilane and partially hydrolyzed oligomer of tetraethoxysilane. The surface treatment which can be used in the present invention is not limited the above described surface treatment and these surface treatments can be used alone or in a combination thereof. Furthermore, the amount of the surface treatment agent relative to the (b) acid-reactive glass powder when performing the surface treatment is not particularly limited, and may be appropriately adjusted depending on the particle diameter of the (b) acid-reactive glass powder and desired property.

Specific examples of the heat treatment of the (b) acid-reactive glass powder include a treatment method which includes heating for a range of 1 hour to 72 hours within a range of 200 °C or more to 800 °C or less using an electric furnace. The heat treatment which can be used in the present invention is not limited the above described treatment and the treatment process may be a processing at uni-temperature or a multi-stage processing at multi-temperatures.

It is preferable that a content of the (b) acid-reactive glass powder is 30 % by mass or more and 75 % by mass or less and more preferably 32 % by mass or more and 73 % by mass or less based on the whole dental resin-reinforced glass ionomer cement composition for luting of the present invention. When the content of the (b) acid-reactive glass powder is less than 30 % by mass, there is a case where the setting is slow and the mechanical characteristic decreases. Furthermore, when the content of (b) acid-reactive glass powder exceeds 75 % by mass there is a case where the kneaded material becomes too hard and the film thickness becomes thick and therefore the bonded and/or adhered dental prosthesis device is lifted to cause discomfort in the bite.

Any polyalkenoic acids can be used without any limitation as the (c) polyalkenoic acid that can be used in the dental resin-reinforced glass ionomer cement composition for luting of the present invention, as long as it is a homopolymer or copolymer of an ethylenically unsaturated monomer having at least one or more acidic groups such as a carboxy group, a phosphate group, a phosphonic acid group and a sulfonic acid group in the molecule such as an ethylenically unsaturated monocarboxylic acid, an ethylenically unsaturated dicarboxylic acid, an ethylenically unsaturated tricarboxylic acid, an ethylenically unsaturated phosphoric acid, an ethylenically unsaturated phosphonic acid and an ethylenically unsaturated sulfonic acid. Further, the (c) polyalkenoic acid may be a copolymer of an ethylenically polymerizable monomer having no acidic group and an ethylenically unsaturated monomer having an acidic group, in the molecule, without any problems. Among these, it is more preferable that the acidic group of the (c) polyalkenoic acid is a carboxy group, because good adhesive property to the tooth substance and high mechanical characteristic can be easily obtained. In addition, in the present specification, "the acidic group of the (c) polyalkenoic acid is a carboxy group" means that 60% or more, preferably 70% or more, and more preferably 80% or more of the acidic groups of the (c) polyalkenoic acid are carboxy groups. The dental resin-reinforced glass ionomer cement composition for luting of the present invention may contain only the (c) polyalkenoic acid where the acidic group of the (c) polyalkenoic acid is a carboxy group as the (c) polyalkenoic acid.

Specific examples of the ethylenically unsaturated monomer having an acidic group that can be used to obtain the (c) polyalkenoic acid include ethylenically unsaturated monocarboxylic acid such as (meth) acrylic acid, 2-chloroacrylic acid, 3-chloroacrylic acid and 2-cyanoacrylic acid; ethylenically unsaturated dicarboxylic acids such as mesaconic acid, maleic acid, maleic anhydride, itaconic acid, itaconic anhydride, fumaric acid, glutaconic acid and citraconic acid; ethylenically unsaturated tricarboxylic acids such as aconitic acid, 1-butene-1,2,4-tricarboxylic acid and 3-butene-1,2,3-tricarboxylic acid; ethylenically unsaturated phosphoric acids such as 2-(meth) acryloyloxyethyl phosphoric acid, 2-(meth) acryloyloxyethylphenyl phosphoric acid, 2-(meth) acryloyloxyethylphenyl-2-bromoethyl phosphoric acid, 10-(meth) acryloyloxydecyl dihydrogen phosphate and 12-(meth) acryloyloxydodecyl dihydrogen phosphate; ethylenically unsaturated phosphonic acids such as 5-(meth) acryloyloxypentyl-3-phosphonopropionate, 6-(meth) acryloyloxyhexyl-3-phosphonopropionate, 10-(meth) acryloyloxydecyl-3-phosphonopropionate, 6-(meth) acryloyloxyhexyl-3-phosphonoacetate and 10-(meth) acryloyloxydecyl-3-phosphonoacetate; and ethylenically unsaturated sulfonic acids such as vinyl sulfonic acid, 2-(meth) acrylamide-2-methylpropanesulfonic acid, 2-sulfoethyl (meth) acrylate, allyl sulfonic acid, methallyl sulfonic acid, methallyloxybenzenesulfonic acid, allyloxybenzenesulfonic acid and styrenesulfonic acid, but are not limited thereto.

The polymerization method used for obtaining various (c) polyalkenoic acid is not particularly limited, and a polymer polymerized by any methods such as solution polymerization, suspension polymerization, emulsion polymerization or the like, may be used without any limitation. In addition, as a polymerization initiator and a chain transfer agent which is used at the time of synthesis of a polymer, a known polymerization initiator and a known chain transfer agent can be used, and the additive amounts thereof may be appropriately adjusted in order to obtain a desired polymer. The (c) polyalkenoic acid obtained by such way can be used alone, or in a combination of a few kinds thereof.

A weight average molecular weight of the (c) polyalkenoic acid is preferably within a range of 3,000 or more and 300,000 or less. Herein, the weight average molecular weight means the average molecular weight which is calculated based on molecular weight distribution measured by gel permeation chromatography. When the weight average molecular weight of the (c) polyalkenoic acid is less than 3,000, there is a case where the removability of excess cement decreases, the adhesive strength decreases or the mechanical characteristic decreases. In addition, when the weight average molecular weight of the (c) polyalkenoic acid exceeds 300,000, there is a case where the removability of excess cement decreases, the working time becomes shorter or the viscosity of the kneaded material increases, resulting in poor kneading property, and therefore it is impossible to obtain a uniform kneaded material to decrease the mechanical characteristic. The dental resin-reinforced glass ionomer cement composition for luting of the present invention may contain only (c) polyalkenoic acid with a weight average molecular weight within a range of 3,000 to 300,000 as the (c) polyalkenoic acid.

In addition, the (c) polyalkenoic acid may be used after some of its acidic groups have been neutralized with a basic compound for the purpose of adjusting the acid-base reactivity with the (b) acid-reactive glass powder as long as a range that various properties are not adversely affected. Examples of the basic compound used for neutralization include alkali metal hydroxides such as sodium hydroxide, potassium hydroxide and lithium hydroxide; alkali metal carbonates such as sodium carbonate, potassium carbonate and lithium carbonate; and alkali metal hydrogen carbonates such as sodium hydrogen carbonate, potassium hydrogen carbonate and lithium hydrogen carbonate. Furthermore, various amine compounds such as primary amines, secondary amines and tertiary amines may be used without any problem as long as the solubility with respect to water after neutralization is maintained. As the amine compound, triethanolamine, diethanolamine, methyldiethanolamine, 2-dimethylaminoethyl (meth) acrylate and the like can be suitably used.

The content of the (c) polyalkenoic acid in the second agent must be within the range of more than 20 % by mass and less than 70 % by mass, preferably more than 25 % by mass and less than 65 % by mass based on the whole of the second agent of the dental resin-reinforced glass ionomer cement composition for luting of the present invention. When the content of the (c) polyalkenoic acid is 20 % by mass or less, the adhesive property to tooth substance and the removability of excess cement decrease. Furthermore, when the content of the (c) polyalkenoic acid is 70 % by mass or more, the adhesive property to tooth substance decreases.

In addition, when the total of the (c) polyalkenoic acid contained in the second agent and the (d) water contained in the second agent described below in the dental resin-reinforced glass ionomer cement composition for luting of the present invention is taken as 100 % by mass, the content of the (c) polyalkenoic acid contained in the second agent must be within the range of more than 45 % by mass and less than 75 % by mass, preferably within the range of more than 50 % by mass and less than 75 % by mass and furthermore preferably within the range of more than 52.5 % by mass and less than 75 % by mass. When the content of the (c) polyalkenoic acid is 45 % by mass or less, the adhesive property to tooth substance and the removability of excess cement decrease. Furthermore, when the content of the (c) polyalkenoic acid is 75 % by mass or more, the adhesive property to tooth substance and the removability of excess cement decrease.

The (d) water that can be used in the dental resin-reinforced glass ionomer cement composition for luting of the present invention is a component which functions as a solvent for dissolving the (c) polyalkenoic acid, diffuses metal ions eluted from the (b) acid-reactive glass powder and induces a cross-linking reaction with the (c) polyalkenoic acid.

Any water can be used as the (d) water as long as it does not contain impurities inhibiting the acid-base reaction and adversely affecting on the settability and the mechanical characteristic of the dental resin-reinforced glass ionomer cement composition for luting of the present invention without any limitations. Specifically, it is preferably to use distilled water or ion-exchanged water.

It is preferable that a content of the (d) water is 6 % by mass or more and 50 % by mass or less based on the whole of the dental resin-reinforced glass ionomer cement composition for luting of the present invention. When the content of the (d) water is less than 6 % by mass or when the content of the (d) water exceeds 50 % by mass, there is a case where that the settability decreases or the mechanical characteristic decreases.

As the (e) polymerizable monomer that can be used in the dental resin-reinforced glass ionomer cement composition for luting of the present invention, any known polymerizable monomer can be used without particularly limitation in terms of its molecular structure. That is, specific examples of a polymerizable unsaturated group contained in the (e) polymerizable monomer include a (meth) acryloyloxy group, a (meth) acrylamide group, a styryl group, a vinyl group and an allyl group, but are not limited thereto. Among these polymerizable unsaturated groups, a (meth) acryloyloxy group and a (meth) acrylamide group are preferable because of its excellent polymerization rate. In addition, there is no particular limitation on the number of polymerizable unsaturated groups contained in the (e) polymerizable monomer. The hydrocarbon group bonded to the polymerizable unsaturated group may be any of an aliphatic hydrocarbon group, an alicyclic hydrocarbon group, an aromatic hydrocarbon group and a combination thereof, and the hydrocarbon group may have any substituent such as an acidic group, a hydroxyl group, a halogen atom, an alkoxy group, an amino group and a glycidyl group. Specific examples of the (e) polymerizable monomer are described below, but not limited thereto. These may be used alone or in a combination of two or more kinds thereof.

Specific examples of the monofunctional polymerizable monomer include (meth) acrylic acid esters such as (meth) acrylic acid, methyl (meth) acrylate, ethyl (meth) acrylate, isopropyl (meth) acrylate, n-propyl (meth) acrylate, isobutyl (meth) acrylate, n-butyl (meth) acrylate, t-butyl (meth) acrylate, sec-butyl (meth) acrylate, n-amyl (meth) acrylate, isoamyl (meth) acrylate, n-hexyl (meth) acrylate, isodecyl (meth) acrylate, lauryl (meth) acrylate, stearyl (meth) acrylate, 2-ethylhexyl (meth) acrylate, cyclohexyl (meth) acrylate, adamantyl (meth) acrylate, phenyl (meth) acrylate, benzyl (meth) acrylate, 2-phenylethyl (meth) acrylate, tetrahydrofurfuryl (meth) acrylate, glycidyl (meth) acrylate, isobornyl (meth) acrylate, allyl (meth) acrylate, 2-methoxyethyl (meth) acrylate, 2-ethoxyethyl (meth) acrylate, phenoxyethyl (meth) acrylate, 2-hydroxyethyl (meth) acrylate, 2-hydroxypropyl (meth) acrylate, 3-hydroxypropyl (meth) acrylate, 2-hydroxybutyl (meth) acrylate, glycerol (meth) acrylate, (meth) acryloyloxyethyl methyl succinate, 2-(meth) acryloyloxyethyl propionate, acetoacetoxyethyl (meth) acrylate, acetoacetoxypropyl (meth) acrylate and acetoacetoxybutyl (meth) acrylate; silane compounds such as γ-(meth) acryloyloxypropyl trimethoxysilane and γ-(meth) acryloyloxypropyl triethoxysilane; amines such as 2-(N,N-dimethylamino) ethyl (meth) acrylate and 2-(N,N-diethylamino) ethyl (meth) acrylate; fluorine-containing (meth) acrylates such as 2,2,2-trifluoroethyl (meth) acrylate, perfluorohexylethyl (meth) acrylate and perfluorooctylethyl (meth) acrylate and (meth) acrylamides thereof; and N-methylol (meth) acrylamide.

Specific examples of the aromatic bifunctional polymerizable monomer include 2,2-bis [4-[3-(meth) acryloyloxy-2-hydroxypropoxy] phenyl]propane, 2,2-bis (4-(meth) acryloyloxyphenyl) propane, 2,2-bis (4-(meth) acryloyloxy ethoxyphenyl) propane, 2,2-bis (4-(meth) acryloyloxy diethoxyphenyl) propane, 2,2-bis (4-(meth) acryloyloxy tetraethoxyphenyl) propane, 2,2-bis (4-(meth) acryloyloxy pentaethoxyphenyl) propane, 2,2-bis (4-(meth) acryloyloxy dipropoxyphenyl) propane, 2-(4-(meth) acryloyloxy ethoxyphenyl)-2-(4-(meth) acryloyloxy diethoxyphenyl) propane, 2-(4-(meth) acryloyloxy diethoxyphenyl)-2-(4-(meth) acryloyloxy triethoxyphenyl) propane, 2-(4-(meth) acryloyloxy dipropoxyphenyl)-2-(4-(meth) acryloyloxy triethoxyphenyl) propane, 2,2-bis (4-(meth) acryloyloxy dipropoxyphenyl) propane, 2,2-bis (4-(meth) acryloyloxy isopropoxyphenyl) propane, 2,2-bis (4-(meth) acryloyloxy polyethoxyphenyl) propane, 9,9-bis [4-(2-(meth) acryloyloxy ethoxy) phenyl] fluorene, and (meth) acrylamides thereof.

Specific examples of the aliphatic bifunctional polymerizable monomer include ethylene glycol di (meth) acrylate, diethylene glycol di (meth) acrylate, triethylene glycol di (meth) acrylate, tetraethylene glycol di (meth) acrylate, polyethylene glycol di (meth) acrylate, dipropylene glycol di (meth) acrylate, tripropylene glycol di (meth) acrylate, neopentyl glycol di (meth) acrylate, 3-methyl-1,5-pentanediol di (meth) acrylate, 1,3-butanediol di (meth) acrylate, 1,4-butanediol di (meth) acrylate, 1,6-hexanediol di (meth) acrylate, 1,9-nonanediol di (meth) acrylate, 1,10-decanediol di (meth) acrylate, tricyclodecane dimethanol di (meth) acrylate, 2-hydroxypropyl-1,3-di (meth) acrylate, 3-hydroxypropyl-1,2-di (meth) acrylate, 2-hydroxy-3-acryloyloxypropyl (meth) acrylate, 1,2-bis (3-(meth) acryloyloxy-2-hydroxypropoxy) ethane, 1,2-bis (3-(meth) acryloyloxy-2-hydroxypropoxy) propane, 2-hydroxy-1,3-bis (3-(meth) acryloyloxy-2-hydroxypropoxy) propane and (meth)acrylamides thereof.

Specific examples of the tri or more functional polymerizable monomer include trimethylolpropane tri (meth) acrylate, trimethylolethane tri (meth) acrylate, trimethylolmethane tri (meth) acrylate, pentaerythritol tri (meth) acrylate, glycerin tri (meth) acrylate, pentaerythritol tetra (meth) acrylate, dipentaerythritol tetra (meth) acrylate, dipentaerythritol penta (meth) acrylate, dipentaerythritol hexa (meth) acrylate, ditrimethylolpropane tetra (meth) acrylate and (meth) acrylamides thereof.

Specific examples of the urethane-based polymerizable monomer include (meth) acrylate compounds having a urethane linkage, which are derived from an adduct of a polymerizable monomer having a hydroxyl group such as 2-hydroxyethyl (meth) acrylate, 2-hydroxypropyl (meth) acrylate and 3-chloro-2-hydroxypropyl (meth) acrylate, and an isocyanate compound such as methylcyclohexane diisocyanate, methylene bis (4-cyclohexyl isocyanate), hexamethylene diisocyanate, trimethylhexamethylene diisocyanate, isophorone diisocyanate, diisocyanate methylmethylbenzene and 4,4-diphenylmethane diisocyanate.

In addition, it is preferable that a content of the (e) polymerizable monomer is 1 % by mass or more and 70 % by mass or less and more preferably 2.5% by mass or more and 60 % by mass or less based on the whole of the dental resin-reinforced glass ionomer cement composition for luting of the present invention. When the content of the (e) polymerizable monomer is less than 1 % by mass or when the content of the (e) polymerizable monomer exceeds 70 % by mass, there is a case where the settability decreases or the mechanical characteristic decreases.

Furthermore, it is preferable that the (e) polymerizable monomer contains a water-soluble polymerizable monomer. In the case of containing a water-soluble polymerizable monomer, there is a case where the wettability of the composition to tooth substance is improved and the adhesive property to tooth substance is improved.

In the present invention, the water-soluble polymerizable monomer means a compound having a solubility of 5 parts by mass or more with respect to 100 g of water at 25 °C and having at least one polymerizable unsaturated group. As the water-soluble polymerizable monomer, it is preferable to have a hydrophilic group such as a hydroxyl group, an oxymethylene group, an oxyethylene group, an oxypropylene group or an amide group.

Specific examples of water-soluble polymerizable monomers include (meth) acrylic acid, 2-hydroxyethyl (meth) acrylate, 2-hydroxypropyl (meth) acrylate, 3-hydroxypropyl (meth) acrylate, 1,3-dihydroxypropyl (meth) acrylate, 2,3-dihydroxypropyl (meth) acrylate, 4-hydroxybutyl (meth) acrylate, 2-((meth) acryloyloxy) ethyltrimethyl ammonium chloride, 3-chloro-2-hydroxypropyl (meth) acrylate, polyethylene glycol di (meth) acrylate (containing 9 or more ethyleneoxy groups), 2-hydroxy-3-acryloyloxypropyl (meth) acrylate, glycerol-1,3-dimethacrylate, N-methylol (meth) acrylamide, N-hydroxyethyl (meth) acrylamide, N,N-bis (2-hydroxyethyl) (meth) acrylamide, N-methoxymethyl (meth) acrylamide, N-ethoxymethyl (meth) acrylamide, diacetone (meth) acrylamide, 4-(meth) acryloyl morpholine, N-trihydroxymethyl-N-methyl (meth) acrylamide, N,N-bis (2-acrylamidoethyl) acrylamide, N,N'-1,2-ethanediylbis{N-[2-(acryloylamino) ethyl] acrylamide} and N-[tris (3-acrylamido propoxymethyl) methyl] acrylamide, but are not limited thereto. These may be used alone or in combination of two or more kinds thereof.

It is preferable that a content of the water-soluble polymerizable monomer is 10 % by mass or more and 97.5 % by mass or less and more preferably 20 % by mass or more and 95 % by mass or less based on the whole of the (e) polymerizable monomer. When the content of the water-soluble polymerizable monomer is less than 10 % by mass, there is a case where the effect of improving adhesive property to tooth substance is not sufficiently exhibited. Furthermore, when the content of the water-soluble polymerizable monomer exceeds 97.5 % by mass, there is a case where the mechanical characteristic decreases.

The dental resin-reinforced glass ionomer cement composition for luting of the present invention may contain (f) acidic component having a molecular weight of 500 or less in order to extend the time where the semi-set state suitable for removing excess cement is maintained. In the present specification, the (f) acidic component having a molecular weight of 500 or less means a compound having an acidic group such as a carboxy group, a phosphoric acid group, a phosphonic acid group and a sulfonic acid group in the molecule and having a molecular weight of 500 or less. Among these of the (f) acidic component having a molecular weight of 500 or less, a compound where the acidic group is a carboxy group is preferable and a compound having two or more carboxy groups is more preferable. The dental resin-reinforced glass ionomer cement composition for luting of the present invention may contain only a compound where the acidic group is a carboxy group and may contain only a compound having two or more carboxy groups, as the (f) acidic component having a molecular weight of 500 or less. It is more preferable that the (f) acidic component having a molecular weight of 500 or less is a compound having no polymerizable group. The dental resin-reinforced glass ionomer cement composition for luting of the present invention may contain only a compound having no polymerizable group as the (f) acidic component having a molecular weight of 500 or less.

Specific examples of the (f) acidic component having a molecular weight of 500 or less include carboxylic acid compounds such as acetic acid, propionic acid, (meth) acrylic acid, 2-chloroacrylic acid, 3-chloroacrylic acid, tartaric acid, mesaconic acid, itaconic acid, maleic acid, fumaric acid, glutaconic acid, citraconic acid, citric acid, malic acid, aconitic acid, tricarballylic acid, 1-butene-1,2,4-tricarboxylic acid, 3-butene-1,2,3-tricarboxylic acid and 4-(meth)acryloyloxyethyl trimellitic acid; phosphoric acid compounds such as phosphoric acid, pyrophosphoric acid, tripolyphosphoric acid, 2-(meth) acryloyloxyethyl phosphoric acid, 2-(meth) acryloyloxyethylphenyl phosphoric acid, 10-(meth) acryloyloxydecyl dihydrogen phosphate and 12-(meth) acryloyl dodecyl dihydrogen phosphate; phosphonic acid compounds such as phosphonic acid, methylphosphonic acid, 5-(meth) acryloyloxypentyl phosphonopropionate, 6-(meth) acryloyloxyhexyl phosphonopropionate, 10-(meth) acryloyloxydecyl phosphonopropionate, 6-(meth) acryloyloxyhexyl phosphonoacetate and 10-(meth)acryloyloxydecyl phosphonoacetate,;sulfonic acid compounds such as vinyl sulfonic acid, allyl sulfonic acid, 2-(meth) acrylamide-2-methylpropane sulfonic acid, 2-sulfoethyl (meth) acrylate, alkylbenzenesulfonic acid and styrenesulfonic acid, but are not limited thereto. These of the (f) acidic component having a molecular weight of 500 or less can be used alone or in a combination of a few kinds thereof.

It is preferable that a content of the (f) acidic component having a molecular weight of 500 or less is within a range of 0.1 % by mass or more and 20 % by mass or less based on the whole dental resin-reinforced glass ionomer cement composition for luting of the present invention. When the content of the (f) acidic component having a molecular weight of 500 or less is less than 0.1 % by mass, there is a case where the time where the semi-set state is maintained is not able to be sufficiently extended. Furthermore, when the content of the (f) acidic component having a molecular weight of 500 or less exceeds 20 % by mass, there is a case where the mechanical characteristic and adhesive property to tooth substance decrease.

The dental resin-reinforced glass ionomer cement composition for luting of the present invention may contain a chemical polymerization initiator other than the (a) aryl borate compound as long as the effect of the present invention are not impaired.

Specific examples of the chemical polymerization initiator other than the (a) aryl borate compound include peroxide/amine compound, peroxide/amine compound/aromatic sulfinic acid or its salt or aromatic sulfonyl compound, peroxide/amine compound/(thio) barbituric acid compound or (thio) barbiturate compound, peroxide/ascorbic acid compound, peroxide/thiourea/vanadium compound or copper compound, (thio) barbituric acid compound/organometallic compound/organohalogen compound, a system combining aromatic sulfinic acid salts or (thio) barbiturate salts which initiate polymerization by the action of an acidic compound and an acidic compound, and an organic boron compound that initiates polymerization by reacting with oxygen or water, but are not limited thereto.

Examples of peroxides include sodium peroxodisulfate, potassium peroxodisulfate, ammonium peroxodisulfate, sodium peroxodiphosphate, potassium peroxodiphosphate, ammonium peroxodiphosphate, diacyl peroxides, alkyl peroxy esters, peroxydicarbonates, monoperoxycarbonates, peroxyketals, dialkyl peroxides, hydroperoxides and ketone peroxides. More specific examples include benzoyl peroxide, p-chlorobenzoyl peroxide, 2,4-dichlorobenzoyl peroxide, diacetyl peroxide, lauroyl peroxide, di-t-butyl peroxide, dicumyl peroxide, cumene hydroperoxide, t-butyl hydroperoxide, 1,1,3,3-tetramethylbutyl hydroperoxide, t-amyl hydroperoxide, iso-propylbenzene hydroperoxide, 5-phenyl-4-pentenyl hydroperoxide, t-butylperoxy isopropyl carbonate, methyl ethyl ketone peroxide, 1,1-bis (t-butylperoxy) cyclohexane, 1,1-bis (t-hexylperoxy) cyclohexane, and t-butylperoxybenzoate, but are not limited thereto.

The amine compound is preferably an aromatic secondary or aromatic tertiary amine, and specific examples thereof include N-methyl-p-toluidine, N-ethyl-p-toluidine, N-(2-hydroxyethyl)-p-toluidine, ethyl p-methyl aminobenzoate, N-methylaniline, N-ethylaniline, N-(2-hydroxyethyl) aniline, N,N-dimethylaniline, N,N-diethylaniline, N,N-bis (2-hydroxyethyl) aniline, N-(2-hydroxyethyl)-N-methylaniline, N-ethyl-N-(2-hydroxyethyl) aniline, N,N-dimethyl-p-toluidine, N,N-diethyl-p-toluidine, N,N-bis (2-hydroxyethyl)-p-toluidine and ethyl p-dimethyl aminobenzoate, but are not limited thereto.

Specific examples of an aromatic sulfinic acid or a salt thereof or an aromatic sulfonyl compound include benzenesulfinic acid, p-toluenesulfinic acid, o-toluenesulfinic acid, 2,4,6-trimethylbenzenesulfinic acid, 2,4,6-triisopropylbenzenesulfinic acid, and its sodium salt, potassium salt, lithium salt or ammonium salt, benzenesulfonyl chloride, benzenesulfonyl fluoride, benzenesulfonamide, benzenesulfonyl hydrazide, p-toluenesulfonyl chloride, p-toluenesulfonyl fluoride, p-toluenesulfonamide and p-toluenesulfonyl hydrazide, but are not limited thereto.

Specific examples of a (thio) barbituric acid compound or a (thio) barbituric acid salt compound include barbituric acid, 1,3-dimethylbarbituric acid, 1,3-diphenylbarbituric acid, 1,5-dimethylbarbituric acid, 5-butyl barbituric acid, 5-ethyl barbituric acid, 5-isopropyl barbituric acid, 5-cyclohexyl barbituric acid, 5-lauryl barbituric acid, 1,3,5-trimethyl barbituric acid, 1,3-dimethyl-5-ethyl barbituric acid, 1,3-dimethyl-n-butyl barbituric acid, 1,3-dimethyl-5-isobutyl barbituric acid, 1,3-dimethyl-5-cyclohexyl barbituric acid, 1,3-dimethyl-5-phenyl barbituric acid, 1-cyclohexyl-5-ethyl barbituric acid, 1-phenyl-5-benzyl barbituric acid, 1-benzyl-5-phenyl barbituric acid, thiobarbituric acid, 1,3-dimethylthiobarbituric acid, 5-phenylthiobarbituric acid and its alkali metal salts (lithium, sodium, potassium salts and the like), alkaline earth metal salts (calcium, strontium, barium salts and the like), ammonium salts, tetramethylammonium salts and tetraethylammonium salts, but are not limited thereto.

Specific examples of ascorbic acid compounds include L(+)-ascorbic acid, isoascorbic acid, L(+)-sodium ascorbate, L(+)-potassium ascorbate, L(+)-calcium ascorbate and sodium isoascorbate, but are not limited thereto.

Specific examples of thiourea compounds include 1,3-dimethylthiourea, tetramethylthiourea, 1,1-diethylthiourea, 1,1,3,3-tetraethylthiourea, 1-allyl-2-thiourea, 1,3-diallylthiourea, 1,3-dibutylthiourea, 1,3-diphenyl-2-thiourea, 1,3-dicyclohexylthiourea, ethylenethiourea, N-methylthiourea, N-phenylthiourea, N-benzoylthiourea and N-acetylthiourea, but are not limited thereto.

Specific examples of vanadium compounds include vanadium acetylacetonate, vanadyl acetylacetonate, vanadyl stearate, vanadium naphthenate and vanadium benzoylacetonate, but are not limited thereto.

Specific examples of the copper compound include copper acetate, copper salicylate, copper gluconate, copper oleate, copper benzoate, acetylacetone copper and copper naphthenate, but are not limited thereto.

Specific examples of the organometallic compound include, in addition to the above copper compound, manganese acetylacetone, manganese naphthenate, manganese octylate, cobalt acetylacetone, cobalt naphthenate, lithium acetylacetone, lithium acetate, zinc acetylacetone, zinc naphthenate, nickel acetylacetone, nickel acetate, aluminum acetylacetone, calcium acetylacetone, iron acetylacetone, chromium acetylacetone and sodium naphthenate, but are not limited to.

Specific examples of the organic halogen compound include tetramethyl ammonium chloride, tetraethyl ammonium chloride, trioctylmethyl ammonium chloride, dilauryl dimethyl ammonium chloride, lauryl dimethyl benzyl ammonium chloride, tetra-n-butyl ammonium chloride, benzyl dimethyl cetyl ammonium chloride and benzyl dimethyl stearyl ammonium chloride, but are not limited thereto.

As the acidic compound to be reacted with the aromatic sulfinates or (thio) barbiturates, any of an inorganic acid and an organic acid can be used without any limitation, but it is preferable to use an organic acid. Furthermore, from the viewpoint of the stability of the set product, it is most preferable to use an organic acid having a polymerizable group, that is, an acidic group-containing polymerizable monomer, as the organic acid. As the acidic group-containing polymerizable monomer, the type and number of polymerizable groups and acidic groups are not particularly limited, and an acidic group-containing polymerizable monomer commonly used in the dental field can be used. Examples of the acidic group include a carboxy group, a phosphoric acid group, a phosphonic acid group and a sulfonic acid group. Representative examples of the acidic group-containing polymerizable monomer used in the dental field include 10-(meth) acryloyloxydecyl dihydrogenphosphate, 6-(meth) acryloyloxyhexyl-3-phosphonoacetate, 4-(meth) acryloyloxyethyl trimellitic acid and its anhydride and 10-(meth) acryloyloxy-1,1-decanedicarboxylic acid, but are not limited thereto. These acidic compounds may be used alone or in a combination of a plurality thereof.

Specific examples of the organic boron compound that initiates polymerization by reacting with oxygen or water include trialkylborons such as triethylboron, tri (n-propyl) boron, triisopropylboron, tri (n-butyl) boron, tri (s-butyl) boron, triisobutylboron, tripentylboron, trihexylboron and tricyclohexylboron, as well as partial oxides thereof, but are not limited thereto.

When the dental resin-reinforced glass ionomer cement composition for luting of the present invention contains a chemical polymerization initiator other than (a) aryl borate compound, it is preferable that a content of the chemical polymerization initiator is 0.01 % by mass or more and 20 % by mass or less and more preferably 0.1 % by mass or more and 15 % by mass or less based on the whole of the (e) polymerizable monomer. The dental resin-reinforced glass ionomer cement composition for luting of the present invention may not contain a chemical polymerization initiator other than the (a) aryl borate compound.

The dental resin-reinforced glass ionomer cement composition for luting of the present invention may contain a photopolymerization initiator that initiates polymerization by ultraviolet or visible light irradiation.

Examples of the photopolymerization initiator that can be used in the dental resin-reinforced glass ionomer cement composition for luting of the present invention includes photosensitizer and photosensitizer/photopolymerization promotor. Specific examples of the photosensitizer include α-diketones such as benzil, camphorquinone, α-naphtil, acetonaphtone, p,p'-dimethoxybenzil, p,p'-dichlorobenzylacetyl, pentadione, 1,2-phenanthrenquinone, 1,4-phenanthrenquinone, 3,4-phenanthrenquinone, 9,10-phenanthrenquinone and naphthoquinone; benzoin alkyl ethers such as benzoin, benzoin methyl ether and benzoin ethyl ether; thioxanthones such as thioxanthone, 2-chlorothioxanthone, 2-methylthioxanthone, 2-isopropylthioxanthone, 2-methoxythioxanthone, 2-hydroxythioxanthone, 2,4-diethylthioxanthone and 2,4-diisopropylthioxanthone; benzophenones such as benzophenone, p-chlorobenzophenone and p-methoxybenzophenone; acylphosphineoxides such as 2,4,6-trimethylbenzoyl diphenylphosphineoxide, bis (2,4,6-trimethylbenzoyl) phenylphosphine oxide, 2,6-dimethoxybenzoyl diphenylphosphine oxide and bis (2,6-dimethoxybenzoyl) phenylphosphine oxide; α-aminoacetophenones such as 2-benzyl-dimethylamino-1-(4-morpholinophenyl)-butanone-1,2-benzyl-diethyl-amino-1-(4-morpholinophenyl) propanone-1; ketals such as benzyldimethylketal, benzyldiethylketal and benzyl (2-methoxyethylketal); coumarins such as 3-(4-methoxybenzoyl) coumarin, 3-benzoyl-5,7-dimethoxycoumarin, 3,3'-carbonyl bis (7-diethylaminocoumarin) and 3,3'-carbonyl bis (7-dibutylaminocoumarin); and titanocenes such as bis (cyclopentadienyl)-bis [2,6-difluoro-3-(1-pyrolyl) phenyl] titanium, bis (cyclopentadienyl)-bis (pentanefluorophenyl) titanium and bis (cyclopentadienyl)-bis (2,3,5,6-tetrafluoro-4-disiloxyphenyl)-titanium, but are not limited thereto.

Specific examples of the photopolymerization promotors include tertiary amines such as N,N-dimethylaniline, N,N-diethylaniline, N,N-di-n-butylaniline, N,N-dibenzylaniline, N,N-dimethyl-p-toluidine, N,N-dimethyl-m-toluidine, N,N-diethyl-p-toluidine, p-bromo-N,N-dimethylaniline, m-chloro-N,N-dimethylaniline, p-dimethylamino benzaldehyde, p-dimethylamino acetophenone, p-dimethylamino benzoic acid, ethyl p-dimethylaminobenzoate, isoamyl p-dimethylaminobenzoate, N,N-dimethylanthranilic acid methyl ester, N,N-dihydroxyethylaniline, N,N-dihydroxyethyl-p-toluidine, p-dimethylaminophenyl alcohol, p-dimethylaminostyrene, N,N-dimethyl-3,5-xylidine, 4-dimethylaminopyridine, N,N-dimethyl-α-naphthylamine, N,N-dimethyl-P-naphthylamine, triethanolamine, tributylamine, tripropylamine, triethylamine, N-methyldiethanolamine, N-ethyldiethanolamine, N,N-dimethylhexylamine, N,N-dimethyldodecylamine, N,N-dimethylstearylamine, N,N-dimethylaminoethyl (meth)acrylate, N,N-diethylaminoethyl (meth)acrylate and 2,2'-(n-butyl imino) diethanol; secondary amines such as N-phenylglycine; barbiturates such as 5-butylbarbituric acid, 1-benzyl-5-phenylbarbituric acid, 1,3,5-trimethylbarbituric acid, sodium 1,3,5-trimethylbarbiturate and calcium 1,3,5- trimethylbarbiturate; triazine compounds such as 2,4,6-tris (trichloromethyl)-s-triazine, 2-phenyl-4,6-bis (trichloromethyl)-s-triazine, 2-(p-chlorophenyl)-4,6-bis (trichloromethyl)-s-triazine and 2-(4-biphenyl)-4,6-bis (trichloromethyl)-s-triazine; diaryliodonium salts such as diphenyliodonium chloride, diphenyliodonium hexafluorophosphate, diphenyliodonium hexafluoroantimonate, diphenyliodonium tetrafluoroborate, diphenyliodonium tetrakis (pentafluorophenyl) borate, bis-(4-methylphenyl) iodonium hexafluorophosphate, bis-(4-methylphenyl) iodonium hexafluoroantimonate, bis-(4-methylphenyl) iodonium tetrakis (pentafluorophenyl) borate, bis (4-tert-butylphenyl) iodonium hexafluoroantimonate, and bis (4-tert-butylphenyl) iodonium tetrakis (pentafluorophenyl) borate; tin compounds such as dibutyltin diacetate, dibutyltin dilaurate, dioctyltin dilaurate, dioctyltin diversate, dioctyltin bis (mercaptoacetic acid isooctyl ester) salt and tetramethyl-1,3-diacetoxydistannoxane; aldehyde compounds such as lauryl aldehyde and terephthalaldehyde; and sulfur-containing compounds such as dodecyl mercaptan, 2-mercaptobenzoxazole, 1-decanethiol and thiosalicylic acid, but are not limited thereto.

In order to enhance photopolymerization promotion performance, it is effective to add, in addition to the above photopolymerization promoter, oxycarboxylic acids such as citric acid, malic acid, tartaric acid, glycolic acid, gluconic acid, α-oxyisobutyric acid, 2-hydroxypropanoic acid, 3-hydroxypropanoic acid, 3-hydroxybutanoic acid, 4-hydroxybutanoic acid and dimethylolpropionic acid, but are not limited thereto.

These polymerization initiators can be used not only singly but also in a combination of a plurality thereof, regardless of the polymerization manner. In addition, there is no problem even if these polymerization initiators are subjected to a secondary treatment such as encapsulation in a microcapsule, if necessary.

When the dental resin-reinforced glass ionomer cement composition for luting of the present invention contains a photopolymerization initiator, it is preferable that a content of the photopolymerization initiator is 0.01 % by mass or more and 20 % by mass or less and more preferably 0.1 % by mass or more and 15 % by mass or less based on the whole of the (e) polymerizable monomer. The dental resin-reinforced glass ionomer cement composition for luting of the present invention may not contain a photopolymerization initiator. In addition, the dental resin-reinforced glass ionomer cement composition for luting of the present invention may not contain a polymerization initiator other than the (a) aryl borate compound.

Further, a non acid-reactive powder can be optionally contained in the dental resin-reinforced glass ionomer cement composition for luting of the present invention to such a range that various properties are not influenced, the purpose of adjusting operability, a mechanical characteristic or a setting characteristic.

As the non acid-reactive powder which can be used in the dental resin-reinforced glass ionomer cement composition for luting of the present invention, any non acid-reactive powder as long as the non acid-reactive powder does not contain an element that reacts with an acid group of the (c) polyalkenoic acid can be used without any limitation. Examples of the non acid-reactive powder include known dental fillers such as an inorganic filler, an organic filler and an organic-inorganic complex filler, and these can be used alone or in a combination of a few kinds thereof. Among them, it is especially preferable that an inorganic filler is used. In addition, a shape of these of the non acid-reactive powder is not particularly limited, and may be any shape such as spherical, needle-like, plate-like, ground-like and scaly-shapes, and aggregate thereof may be used without any problems. An average particle diameter of the non acid-reactive powder is not particular limited, but may be preferably 0.001 µm or more and 30 µm or less.

Specific examples of the inorganic filler include quartz, amorphous silica, ultrafine silica, various glasses which does not contain element which may react with an acid group (including a glass by melting method, synthetic glass by sol-gel method, a glass produced by a vapor phase reaction, and the like), silicon nitride, silicon carbide and boron carbide, but is not limited thereto.

When the non acid-reactive powder is contained in the dental resin-reinforced glass ionomer cement composition for luting of the present invention, it is preferable that the content of the non acid-reactive powder is 0.001 % by mass or more and 20 % by mass or less based on the wholefo the composition. The dental resin-reinforced glass ionomer cement composition for luting of the present invention may not contain a non acid-reactive powder.

Furthermore, when the dental resin-reinforced glass ionomer cement composition for luting of the present invention includes a paste form, a thickener can be optionally contained to such a range that various properties are not adversely affected, for the purpose of adjusting the paste property.

As the thickener which can be used in the dental resin-reinforced glass ionomer cement composition for luting of the present invention, any of an inorganic thickener and an organic thickener may be used. Specific examples of an inorganic thickener include fumed silica, calcium carbonate, calcium silicate, magnesium silicate, and a clay mineral such as saponite, montmorillonite, beidellite, vermiculite, sauconite, stevensite, hectorite, smectite, nekutaito and sepiolite.

Specific examples of an organic thickener include methyl cellulose, hydroxyethyl cellulose, hydroxymethyl cellulose, hydroxypropyl cellulose, carboxymethyl cellulose, carboxymethyl cellulose sodium, carboxymethyl cellulose calcium, carboxypolymethylene, sodium alginate, propylene glycol alginate ester, sodium polyacrylate, starch, starch sodium glycolate, starch phosphate ester, polyvinyl pyrrolidone, carboxyvinyl polymer, khaya gum, arabic gum, karaya gum and guar gum. These photopolymerization accelerator may be used alone or as a mixture of two or more thereof.

When the thickener is contained in the dental resin-reinforced glass ionomer cement composition for luting of the present invention, it is preferable that the content of the thickener in the paste is within a range of 0.001 % by mass or more and 10.0 % by mass or less. The dental resin-reinforced glass ionomer cement composition for luting of the present invention may not contain a thickener.

The dental resin-reinforced glass ionomer cement composition for luting of the present invention may arbitrarily contain a surfactant, a preservative, an antibacterial agent, a colorant, a fluorescent agent, an inorganic fiber material, an organic fiber material and other conventionally known additives. The dental resin-reinforced glass ionomer cement composition for luting of the present invention may consists of a first agent containing the (a) aryl borate compound and the (b) acid-reactive glass powder and a second agent containing the (c) polyalkenoic acid and the (d) water, and at least one of the first agent and the second agent may contain only the (e) polymerizable monomer and one or more of the components specifically described in the present specification. The dental resin-reinforced glass ionomer cement composition for luting of the present invention may contain only a powder material containing the (a) aryl borate compound and the (b) acid-reactive glass powder, and the (c) polyalkenoic acid, the (d) water, the (e) polymerizable monomer and the (f) acidic component having a molecular weight of 500 or less. The dental resin-reinforced glass ionomer cement composition for luting of the present invention may contain only a powder material containing the (a) aryl borate compound and the (b) acid-reactive glass powder, and the (c) polyalkenoic acid, the (d) water, the (e) polymerizable monomer, the (f) acidic component having a molecular weight of 500 or less and one or more of the components specifically described in the present specification.

The dental resin-reinforced glass ionomer cement composition for luting of the present invention may be provided in any form, such as powder material/liquid material, paste/liquid material, or paste/paste, so long as the (a) aryl borate compound and/or the (b) acid-reactive glass powder do not coexist with the (c) polyalkenoic acid and/or the (f) acidic component having a molecular weight of 500 or less in the presence of the (d) water.

Specific examples of the form of the powder material/liquid material include a combination of a powder material containing the (a) aryl borate compound and the (b) acid-reactive glass powder and a liquid material containing the (c) polyalkenoic acid, the (d) water, the (e) polymerizable monomer and the (f) acidic component having a molecular weight of 500 or less, a combination of a powder material containing the (a) aryl borate compound, the (b) acid-reactive glass powder and the (f) acidic component having a molecular weight of 500 or less and a liquid material containing the (c) polyalkenoic acid, the (d) water and the (e) polymerizable monomer, a combination of a powder material containing the (a) aryl borate compound, the (b) acid-reactive glass powder, the (c) polyalkenoic acid and the (f) acidic component having a molecular weight of 500 or less and a liquid material containing the (c) polyalkenoic acid, the (d) water and the (e) polymerizable monomer, a combination of a powder material containing the (a) aryl borate compound, the (b) acid-reactive glass powder and the (c) polyalkenoic acid and a liquid material containing the (c) polyalkenoic acid, the (d) water, the (e) polymerizable monomer and the (f) acidic component having a molecular weight of 500 or less, but are not limited thereto.

Specific examples of the form of the paste/liquid material include a combination of a paste containing the (a) aryl borate compound, the (b) acid-reactive glass powder and the (e) polymerizable monomer and a liquid material containing the (c) polyalkenoic acid, the (d) water and the (f) acidic component having a molecular weight of 500 or less, a combination of a paste containing the (a) aryl borate compound, the (b) acid-reactive glass powder and the (e) polymerizable monomer and a liquid material containing the (c) polyalkenoic acid, the (d) water, the (e) polymerizable monomer and the (f) acidic component having a molecular weight of 500 or less, a combination of a paste containing the (a) aryl borate compound, the (b) acid-reactive glass powder and the (d) water and a liquid material containing the (c) polyalkenoic acid, the (d) water, the (e) polymerizable monomer and the (f) acidic component having a molecular weight of 500 or less, a combination of a paste containing the (a) aryl borate compound, the (b) acid-reactive glass powder, the (d) water and the (e) polymerizable monomer and a liquid material containing the (c) polyalkenoic acid, the (d) water and the (f) acidic component having a molecular weight of 500 or less, and a combination of a paste containing the (a) aryl borate compound, the (b) acid-reactive glass powder, the (d) water and the (e) polymerizable monomer and a liquid material containing the (c) polyalkenoic acid, the (d) water, the (e) polymerizable monomer and the (f) acidic component having a molecular weight of 500 or less, but are not limited thereto.

Specific examples of the form of the paste/paste include a combination of a first paste containing the (a) aryl borate compound, the (b) acid-reactive glass powder and the (e) polymerizable monomer and a second paste containing the (c) polyalkenoic acid, the (d) water and the (f) acidic component having a molecular weight of 500 or less, a combination of a first paste containing the (a) aryl borate compound, the (b) acid-reactive glass powder and the (e) polymerizable monomer and a second paste containing the (c) polyalkenoic acid, the (d) water, the (e) polymerizable monomer and the (f) acidic component having a molecular weight of 500 or less, a combination of a first paste containing the (a) aryl borate compound, the (b) acid-reactive glass powder and the (d) water and a second paste containing the (c) polyalkenoic acid, the (d) water, the (e) polymerizable monomer and the (f) acidic component having a molecular weight of 500 or less, a combination of a first paste containing the (a) aryl borate compound, the (b) acid-reactive glass powder, the (d) water and the (e) polymerizable monomer and a second paste containing the (c) polyalkenoic acid, the (d) water and the (f) acidic component having a molecular weight of 500 or less, and a combination of a first paste containing the (a) aryl borate compound, the (b) acid-reactive glass powder, the (d) water and the (e) polymerizable monomer and a second paste containing the (c) polyalkenoic acid, the (d) water, the (e) polymerizable monomer and the (f) acidic component having a molecular weight of 500 or less, but are not limited thereto.

### [Example]

The present invention is described in more detail and specifically with reference to Examples. However, the present invention is not limited to Examples. The components (a) to (f) and other components used for manufacturing the dental resin-reinforced glass ionomer cement compositions for luting of Examples and Comparative Examples, and their abbreviations are as follows.

### [(a) Aryl borate compound]

### <Aryl borate compound having 4 aryl groups in one molecule >

· TPBNa: Sodium tetraphenylborate
· TPBK: Potassium tetraphenylborate

### <Aryl borate compound having 3 aryl groups in one molecule >

· BTPB: 1,2-dicyclohexyl-4,4,5,5-tetramethyl biguanidinium = n-butyl triphenylborate

### [(b) Acid-reactive glass powder]

· CK-Si-1: Silane-treated fluoroaluminosilicate glass powder 1 (50% particle diameter: 0.7 µm)
· CK-Si-2: Silane-treated fluoroaluminosilicate glass powder 2 (50% particle diameter: 2.3 µm)

### [(c) Polyalkenoic acid]

### <Polyalkenoic acid having carboxy group>

· PCA1: acrylic acid homopolymer powder (weight average molecular weight: 30,000)
· PCA2: acrylic acid-3-butene-1,2,3-tricarboxylic acid copolymer powder (weight average molecular weight: 80,000)
· PCA3: acrylic acid homopolymer powder (weight average molecular weight: 1,000)
· PCA4: acrylic acid homopolymer powder (weight average molecular weight: 350,000)

### <Polyalkenoic acid having no carboxy group>

· PVSA: polyvinyl sulfonic acid powder (weight average molecular weight: 10,000)

### [(d) Water]

· Distilled water

### [(e) Polymerizable monomer]

· Bis-GMA: 2,2-bis[4-[3-methacryloyloxy-2-hydroxypropoxy] phenyl] propane
· UDMA: urethane dimethacrylate
· 3G: Triethylene glycol dimethacrylate
· HEMA: 2-hydroxyethyl methacrylate
· GDMA: glycerol-1,3-dimethacrylate
· FOM-03006: N-[tris (3-acrylamido propoxymethyl) methyl] acrylamide

### [(f) Acidic component having a molecular weight of 500 or less]

### <Acidic component having two or more carboxy groups and no polymerizable group>

· tartaric acid
· citric acid
· malic acid

### <Acidic component having one carboxy group and no polymerizable group>

· propionic acid

### <Acidic component having two or more carboxy groups and a polymerizable group>

· 4-AET: 4-acryloyloxyethyl trimellitic acid

### <Acidic component having no carboxy group and a polymerizable group>

· 10-MDP: 10-methacryloyloxydecyl dihydrogen phosphate

### [Others]

· BPO: benzoyl peroxide
· KPS: potassium peroxodisulfate
· DMBE: ethyl p-dimethylaminobenzoate
AA: ascorbic acid
· p-TSNa: sodium p-toluenesulfinate
· Aerosil R711: fumed silica (average primary particle diameter: about 12 nm)
· Aerosil R8200: fumed silica (average primary particle diameter: about 12 nm)
· Aerosil #200: fumed silica (average primary particle diameter: about 12 nm)

The manufacturing method of the silane-treated fluoroaluminosilicate glass powders 1 and 2 are as follows.

### [Manufacture of fluoroaluminosilicate glass powder 1]

Various raw materials: silicon dioxide, aluminum oxide, aluminum phosphate, sodium fluoride and strontium carbonate (glass composition: SiO₂: 26.4 wt.%, Al₂O₃: 29.3 wt.%, SrO: 20.5 wt.%, P₂O₅: 10.9 wt.%, Na₂O: 2.5 wt.%, and F: 10.4 wt.%) were mixed and the mixed raw material was molten at 1600 °C in a melting furnace. The molten liquid was taken out from the melting furnace and was quenched in water to manufacture a glass. The resulting glass was pulverized until the 50% particle diameter became 0.7 µm to obtain fluoroaluminosilicate glass powder 1. The 50% particle diameter was measured by a laser diffraction type grain size measuring apparatus (Microtrac MT3300EXII: NIKKISO Co., Ltd.).

### [Manufacture of fluoroaluminosilicate glass powder 2]

Various raw materials: silicon dioxide, aluminum oxide, aluminum phosphate, sodium fluoride and strontium carbonate (glass composition: SiO₂: 27.0 wt.%, Akl₂O₃: 32.5 wt.%, SrO: 12.8 wt.%, P₂O₅: 10.4 wt.%, Na₂O: 4.5 wt.%, and F: 12.8 wt.%) were mixed and the mixed raw material was molten at 1600 °C in a melting furnace. The molten liquid was taken out from the melting furnace and was quenched in water to manufacture a glass. The resulting glass was pulverized until the 50% particle diameter became 2.3 µm to obtain fluoroaluminosilicate glass powder 2. The 50% particle diameter was measured by a laser diffraction type grain size measuring apparatus (Microtrac MT3300EXII: NIKKISO Co., Ltd.).

### [Silane treatment of fluoroaluminosilicate glass powders 1 and 2]

The fluoroaluminosilicate glass powder 1 or 2 of 200 g was dispersed in 500 mL of water, 2g of 3-methacryloyloxypropyltrimethoxysilane was added, and the mixture was stirred at room temperature for 2 hours. After removing the solvent, it was further dried at 100 °C for 5 hours to obtain silane-treated fluoroaluminosilicate glass powders 1 and 2 (CK-Si-1 and 2).

### [Manufacture of paste material]

The components were mixed in the ratios shown in Tables 1 to 3 to manufacture the first agents (paste) and the second agents (paste) of the dental resin-reinforced glass ionomer cement compositions for luting of Examples and Comparative Examples.

### [Manufacture of powder material and liquid material]

The components were mixed in the ratios shown in Tables 4 to 6 to manufacture the first agents (powder material) and the second agents (liquid material) of the dental resin-reinforced glass ionomer cement compositions for luting of Examples and Comparative Examples.

### Composition of the first agent (paste) used in Examples and Comparative examples (% by mass)

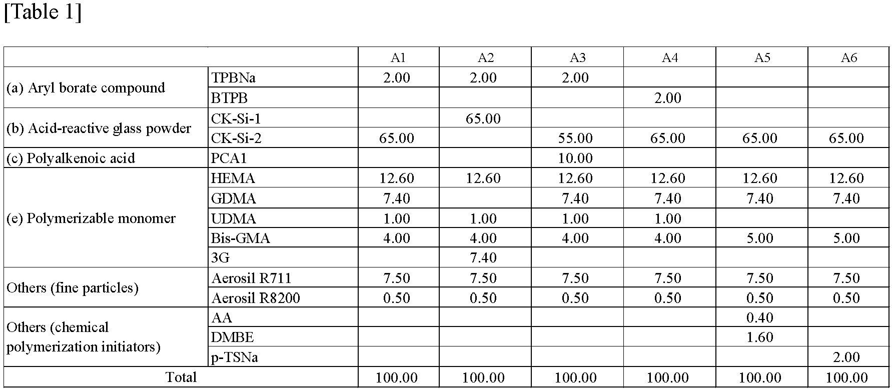

### Composition of the second agent (paste) used in Examples and Comparative examples (% by mass)

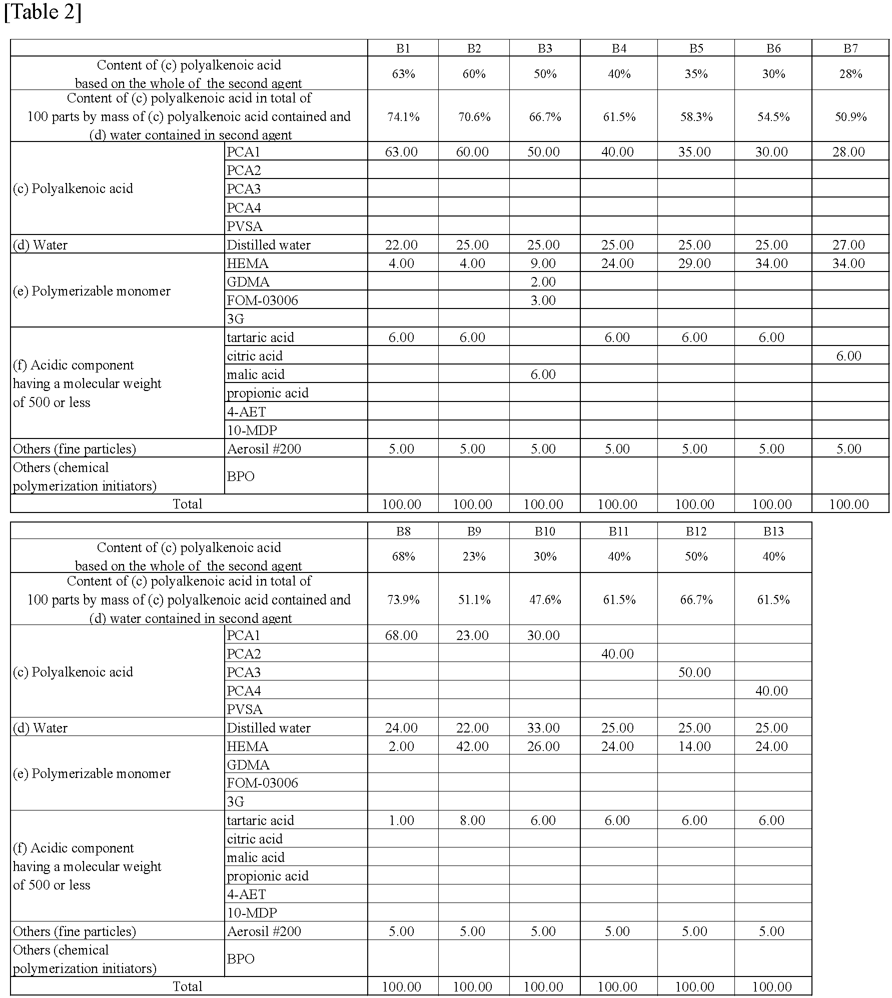

### Composition of the second agent (paste) used in Examples and Comparative examples (% by mass)

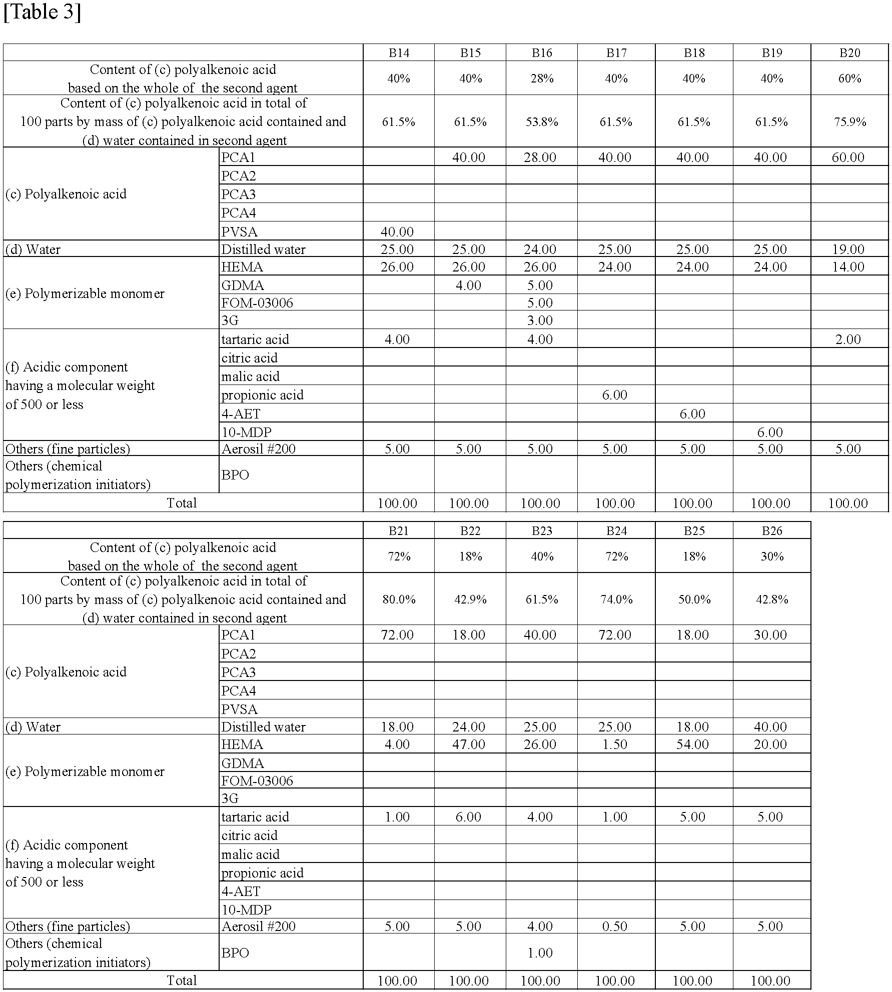

### Composition of the first agent (powder material) used in Examples and Comparative examples (% by mass)

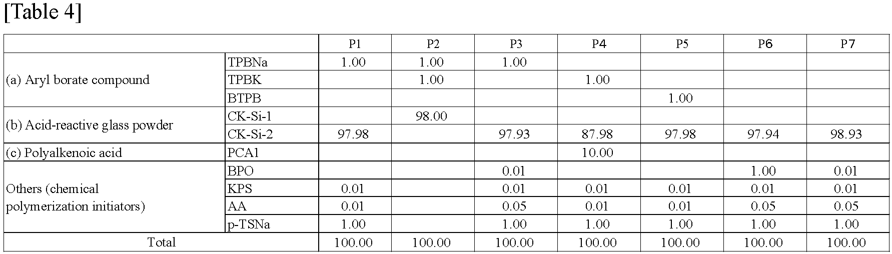

### Composition of the second agent (liquid material) used in Examples and Comparative examples (% by mass)

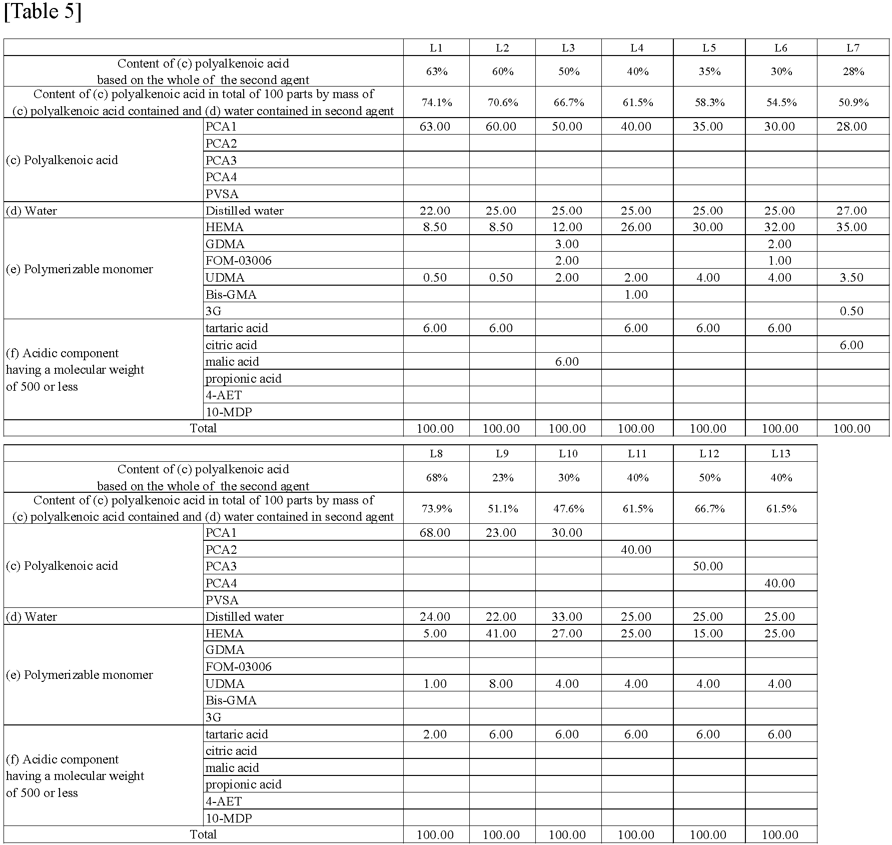

### Composition of the second agent (liquid material) used in Examples and Comparative examples (% by mass)

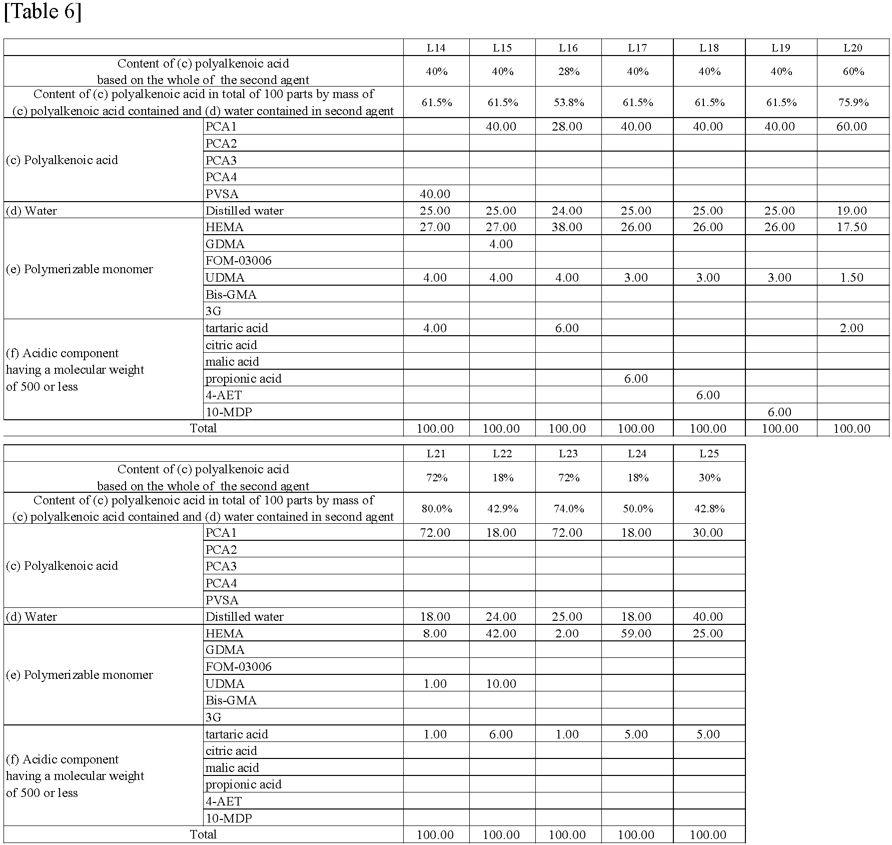

Excess cement removability and tensile adhesive strength of the dental resin-reinforced glass ionomer cement compositions for luting manufactured by mixing these first agents and the second agents in the combinations and mass ratios shown in Tables 7 to 10 (Examples 1 to 66 and Comparative Examples 1 to 30) were evaluated. The evaluation methods are as follows.

### [Evaluation of excess cement removability]

The first agents and the second agents corresponding to the dental resin-reinforced glass ionomer cement composition for luting of the Examples or Comparative Examples were kneaded in the proportions shown in Tables 7 to 10 for 20 seconds in paste/paste form or for 30 seconds in powder/liquid form, and 0.3 g of the kneaded material was then applied to the center of the top surface of a resin block (12 mm long x 16 mm wide x 10 mm high). A resin block of the same shape was pressed firmly against the surface applied with the kneaded material vertically so that the sides of the block were the same length and overlapped, causing the kneaded material to protrude from between the resin blocks. Thereafter, the resin blocks were allowed to stand at 23 °C and then after 2 minute, after 3 minute and after 4 minute from the start of kneading, the kneaded material that protruded from between the resin blocks was removed as excess cement using an instrument (thin) (MiCD instrument #2 (the diameter ϕ of the tip is 0.3 mm, manufactured by SHOFU INC.)) or an instrument (thick) (MiCD instrument #4 (the diameter ϕ of the tip is 1.5 mm, manufactured by SHOFU INC.)) and excess cement removability was evaluated according to the following evaluation criteria. When the evaluation result was A or B, it was determined that the cement was in a semi-set state suitable for removing excess cement. Furthermore, when the evaluation results of after 2 minute, after 3 minute and after 4 minute from the start of kneading were consecutively A or B (that is, when the evaluation results of after 2 minute and after 3 minute, after 3 minute and after 4 minute, or after 2 minute, after 3 minute and after 4 minute were a combination of A and A, a combination of A and B, a combination ofB and A, or a combination ofB and B), it was determined to have good excess cement removability. In the case of such a combination of consecutive evaluations, A was assigned 2 points and B was assigned 1 point, and it was determined that the higher the total score, the better the excess cement removability.

### [Evaluation Criteria]

A: The excess cement was in a semi-set state and it was possible to easily remove the excess cement in one lump using the instrument (thin) (MiCD instrument #2, the diameter ϕ of the tip is 0.3 mm).
B: The excess cement was in a semi-set state. When the instrument (thin) (MiCD instrument #2, the diameter ϕ of the tip is 0.3 mm) was used, the excess cement was torn off and it was impossible to remove the excess cement in one lump. When the instrument (thick) (MiCD instrument #4, the diameter ϕ of the tip is 1.5 mm) was used, it was possible to easily remove the excess cement in one lump.
C: The excess cement was in a semi-set state, but was easily torn off. It was impossible to remove the excess cement in one lump even using the instrument (thick) (MiCD instrument #4, the diameter ϕ of the tip is 1.5 mm), but it was possible to remove the excess cement.
D: The excess cement was set and it was difficult to remove the excess cement even using the instrument (thick) (MiCD instrument #4, the diameter ϕ of the tip is 1.5 mm).
E: The excess cement was not set and it was difficult to remove the excess cement even using the instrument (thick) (MiCD instrument #4, the diameter ϕ of the tip is 1.5 mm).

### [Tensile adhesive strength test]

A test specimen manufactured by embedding a bovine anterior tooth in epoxy resin was polished with water-resistant abrasive paper #600 to expose the enamel surface. On the other hand, an adhesion surface of a cylindrical stainless rod (ϕ4.5 mm) was subjected to sandblasting treatment (0.2 MPa) with alumina particles (average particle diameter 50 µm), and then washed with water and dried. The first agent and the second agent corresponding to the dental resin-reinforced glass ionomer cement composition for luting of the Examples or Comparative Examples were mixed in the ratios shown in Tables 7 to 10 for 20 seconds in paste/paste form or for 30 seconds in powder/liquid form, and then an appropriate amount of the kneaded material was applied to the adhesion surface of a stainless rod and pressed against the polished enamel surface. With a load of 10 N applied to the upper surface of the stainless rod, excess cement was removed, and the test specimen was allowed to set for 5 minutes in an environment of 37 °C and a humidity of 90 % or higher. After setting, the load of 10 N was removed, and the test specimen was allowed to stand for 1 hour in an environment of 37 °C and a humidity of 90 % or more, and then immersed in 37 °C water for 24 hours to obtain an adhesive test specimen. An Instron universal tester (manufactured by Instron Corporation) was used to measure the tensile adhesive strength of the manufactured adhesive test specimen at a crosshead speed of 75 mm/min.

### [Evaluation Criteria]

In the case of 6 MPa or more, it was determined that an adhesive strength was sufficiently usable and excellent.

In the case of 4 MPa or more and less than 6 MPa, it was determined that an adhesive strength was usable.

In the case of less than 4 MPa, it was determined that an adhesive strength was unsuitable for use.

### Evaluation results

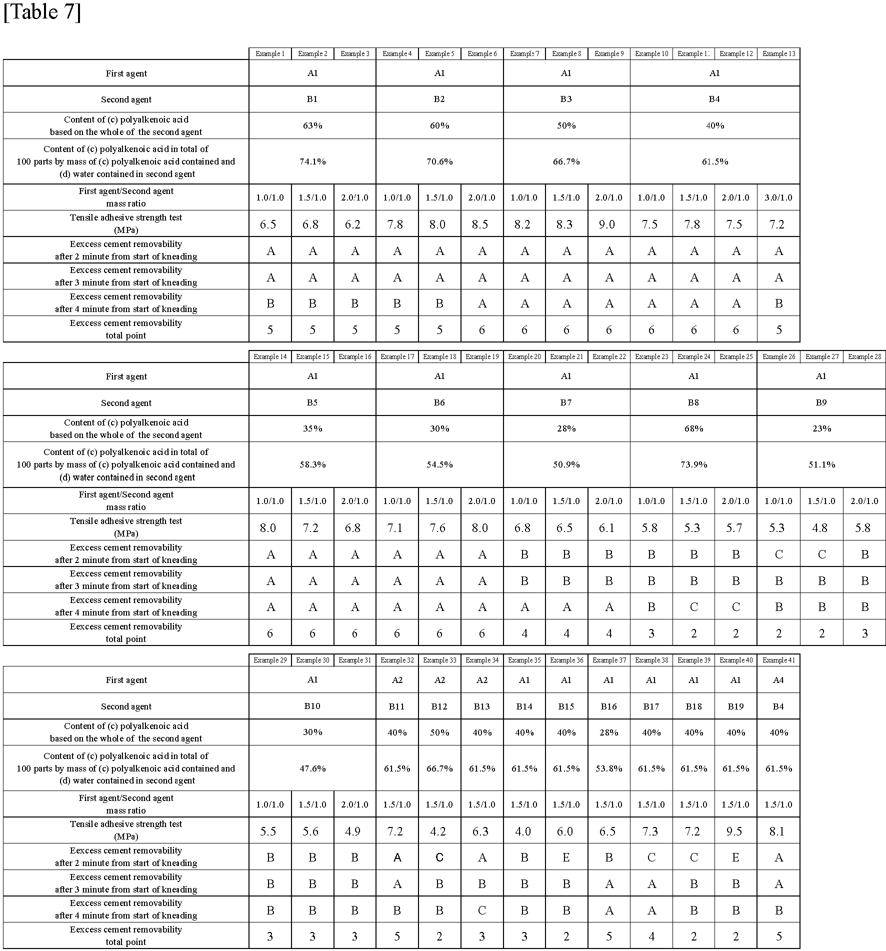

### Evaluation results

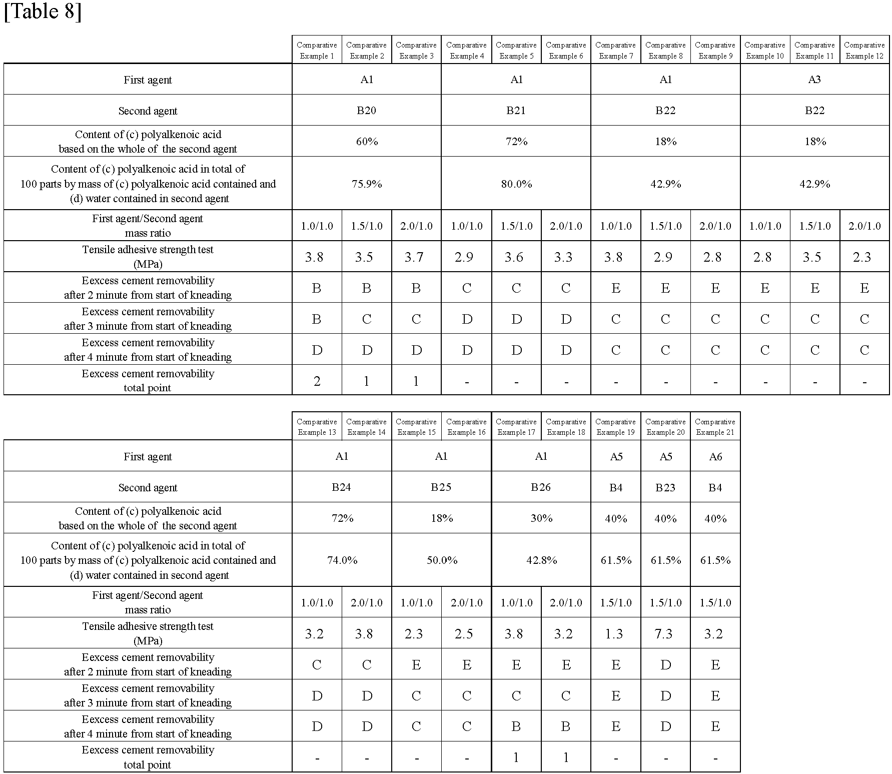

### Evaluation results

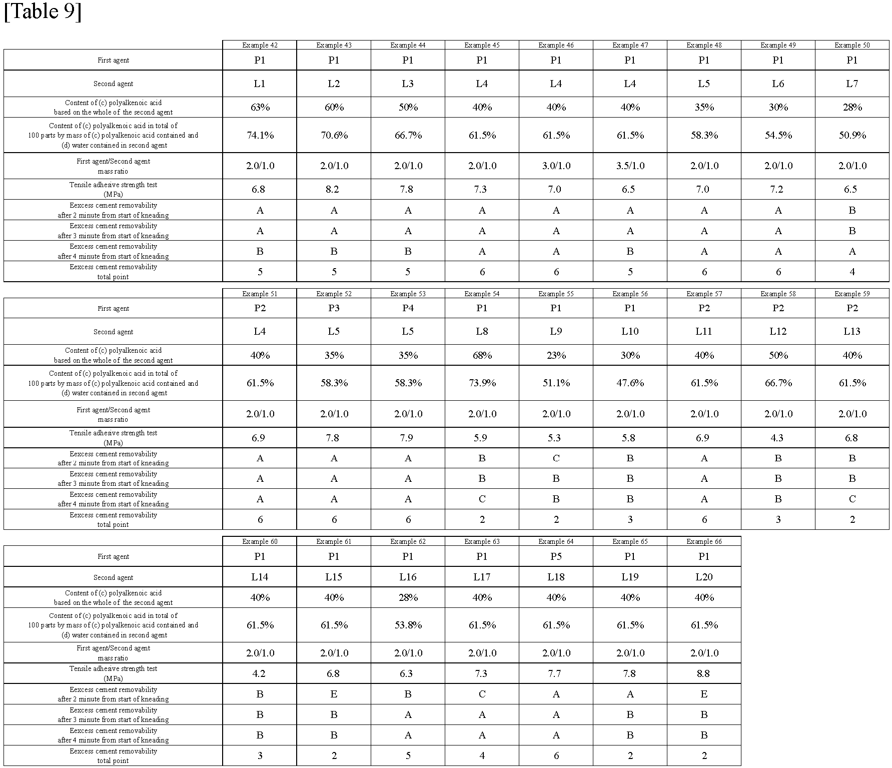

### Evaluation results

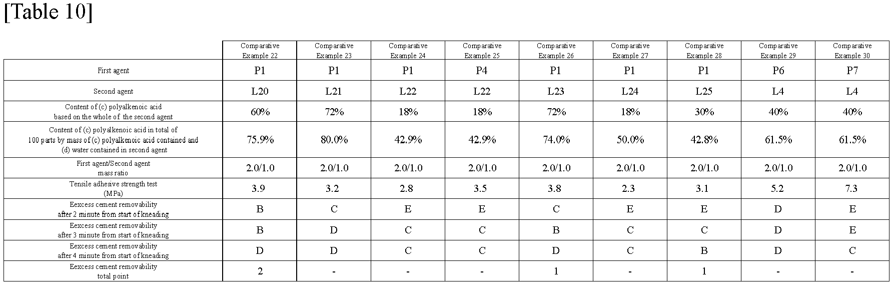

### <Examples 1 to 66>

Examples 1 to 66 had good excess cement removability. Furthermore, these exhibited sufficiently usable and excellent adhesive property to the tooth substance, and had desirable properties as a dental resin-reinforced glass ionomer cement for luting.

### <Comparative Examples 1 to 3>

In the second agents used in Comparative Examples 1 to 3, the proportion of the (c) polyalkenoic acid was large in the ratio of the (c) polyalkenoic acid to the (d) water contained in the second agent. As a result of evaluating Comparative Example 1, it was found that the adhesive property to tooth substance was low. Furthermore, as a result of evaluating Comparative Examples 2 and 3, it was found that the removability of excess cement was poor and the adhesive property to tooth substance was also low.

### <Comparative Examples 4 to 6>

In the second agents used in Comparative Examples 4 to 6, the content of the (c) polyalkenoic acid was large. Furthermore, in the ratio of the (c) polyalkenoic acid to the (d) water contained in the second agent, the proprotion of the (c) polyalkenoic acid was large. As a result of evaluating Comparative Examples 4 to 6, it was found that the removability of excess cement was poor and the adhesive property to tooth substance was also low.

### <Comparative Examples 7 to 9>

In the second agents used in Comparative Examples 7 to 9, the content of the (c) polyalkenoic acid was small. Furthermore, in the ratio of the (c) polyalkenoic acid to the (d) water contained in the second agent, the proportion of the (c) polyalkenoic acid was small. As a result of evaluating Comparative Examples 7 to 9, it was found that the removability of excess cement was poor and the adhesive property to tooth substance was also low.

### <Comparative Examples 10 to 12>

In the second agents used in Comparative Examples 10 to 12, the content of the (c) polyalkenoic acid was small. Furthermore, in the ratio of the (c) polyalkenoic acid to the (d) water contained in the second agent, the proportion of the (c) polyalkenoic acid was small. On the other hand, the first agents used in Comparative Examples 10 to 12 contained the (c) polyalkenoic acid to compensate for the lack of the (c) polyalkenoic acid in the second agent. However, as a result of evaluating Comparative Examples 10 to 12, it was found that the removability of excess cement was poor and the adhesive property to tooth substance was also low.

### <Comparative Examples 13 and 14>

In the second agents used in Comparative Examples 13 and 14, the content of the (c) polyalkenoic acid was large. As a result of evaluating Comparative Examples 13 and 14, it was found that the removability of excess cement was poor and the adhesive property to tooth substance was also low.

### <Comparative Examples 15 and 16>

In the second agents used in Comparative Examples 15 and 16, the content of the (c) polyalkenoic acid was small. As a result of evaluating Comparative Examples 15 and 16, it was found that the removability of excess cement was poor and the adhesive property to tooth substance was also low.

### <Comparative Examples 17 and 18>

In the second agents used in Comparative Examples 17 to 18, the proportion of the (c) polyalkenoic acid was small in the ratio of the (c) polyalkenoic acid to the (d) water contained in the second agent. As a result of evaluating Comparative Examples 17 to 18, it was found that the removability of excess cement was poor and the adhesive property to tooth substance was also low.

### <Comparative Example 19>

Comparative Example 19 did not contain the (a) aryl borate compound, but contained AA and DMBE as other chemical polymerization initiators. As a result of evaluating Comparative Example 19, it was found that the removability of excess cement was poor and the adhesive property to tooth substance was also low.

### <Comparative Example 20>

Comparative Example 20 did not contain the (a) aryl borate compound, but contained BPO, AA and DMBE as other chemical polymerization initiators. As a result of evaluating Comparative Example 20, it was found that the removability of excess cement was poor.

### <Comparative Example 21>

Comparative Example 21 did not contain the (a) aryl borate compound, but contained p-TSNa as other chemical polymerization initiators. As a result of evaluating Comparative Example 21, it was found that the removability of excess cement was poor and the adhesive property to tooth substance was also low.

### <Comparative Example 22>

In the second agent used in Comparative Example 22, the proportion of the (c) polyalkenoic acid was large in the ratio of the (c) polyalkenoic acid to the (d) water contained in the second agent. As a result of evaluating Comparative Example 22, it was found that the adhesive property to tooth substance was low.

### <Comparative Example 23>

In the second agent used in Comparative Example 23, the content of the (c) polyalkenoic acid was large. Furthermore, in the ratio of the (c) polyalkenoic acid to the (d) water contained in the second agent, the proportion of the (c) polyalkenoic acid was large. As a result of evaluating Comparative Example 23, it was found that the removability of excess cement was poor and the adhesive property to tooth substance was also low.

### <Comparative Example 24>

In the second agent used in Comparative Example 24, the content of the (c) polyalkenoic acid was small. Furthermore, in the ratio of the (c) polyalkenoic acid to the (d) water contained in the second agent, the ratio of the (c) polyalkenoic acid was small. As a result of evaluating Comparative Example 24, it was found that the removability of excess cement was poor and the adhesive property to tooth substance was also low.

### <Comparative Example 25>

In the second agent used in Comparative Example 25, the content of the (c) polyalkenoic acid was small. Furthermore, in the ratio of the (c) polyalkenoic acid to the (d) water contained in the second agent, the ratio of the (c) polyalkenoic acid was small. On the other hand, the first agent used in Comparative Example 25 contained the (c) polyalkenoic acid to compensate for the lack of the (c) polyalkenoic acid in the second agent. However, as a result of evaluating Comparative Example 25, it was found that the removability of excess cement was poor and the adhesive property to tooth substance was also low.

### <Comparative Example 26>

In the second agent used in Comparative Example 26, the content of the (c) polyalkenoic acid was large. As a result of evaluating Comparative Example 26, it was found that the removability of excess cement was poor and the adhesive property to tooth substance was also low.

### <Comparative Example 27>

In the second agent used in Comparative Example 27, the content of the (c) polyalkenoic acid was small. As a result of evaluating Comparative Example 27, it was found that the removability of excess cement was poor and the adhesive property to tooth substance was also low.

### <Comparative Example 28>

In the second agent used in Comparative Example 28, the proportion of the (c) polyalkenoic acid was small even in the ratio of the (c) polyalkenoic acid to the (d) water contained in the second agent. As a result of evaluating Comparative Example 28, it was found that the removability of excess cement was poor and the adhesion to tooth structure was also low.

### <Comparative Example 29>

Comparative Example 29 did not contain the (a) aryl borate compound, but contained BPO (1 % by mass), KPS, AA and p-TSNa as other chemical polymerization initiators. As a result of evaluating Comparative Example 29, it was found that the removability of excess cement was poor.

### <Comparative Example 30>

Comparative Example 30 did not contain the (a) aryl borate compound, but contained BPO (0.01 % by mass), KPS, AA and p-TSNa as other chemical polymerization initiators. As a result of evaluating Comparative Example 30, it was found that the removability of excess cement was poor.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this disclosure without difficulty. Accordingly, any such changes and modifications are intended to be included in the scope disclosure the embodiments.

### Industrial applicability

The dental resin-reinforced glass ionomer cement composition for luting of the present invention is useful as a dental cement because it exhibits excellent adhesive property to tooth substance and can maintain a semi-set state suitable for removing excess cement for a long period during the set process, and the excess cement in the semi-set state is difficult to tear.

## Claims

1. A dental resin-reinforced glass ionomer cement composition for luting consisting of a first agent including (a) aryl borate compound and (b) acid-reactive glass powder and a second agent including (c) polyalkenoic acid and (d) water, wherein
at least one of the first agent and the second agent contains (e) polymerizable monomer,
the content of the (c) polyalkenoic acid contained in the second agent based on the whole of the second agent is more than 20 % by mass and less than 70 % by mass, and the content of the (c) polyalkenoic acid contained in the second agent in the total of 100 parts by mass of the (c) polyalkenoic acid contained in the second agent and the (d) water contained in the second agent is more than 45 parts by mass and less than 75 parts by mass.

2. The dental resin-reinforced glass ionomer cement composition for luting according to claim 1, wherein
the content of the (c) polyalkenoic acid contained in the second agent based on the whole of the second agent is more than 25 % by mass and less than 65 % by mass.

3. The dental resin-reinforced glass ionomer cement composition for luting according to claim 1 or 2, wherein
the content of the (c) polyalkenoic acid contained in the second agent in the total of 100 parts by mass of the (c) polyalkenoic acid contained in the second agent and the (d) water contained in the second agent is more than 50 parts by mass and less than 75 parts by mass.

4. The dental resin-reinforced glass ionomer cement composition for luting according to claim 1 or 2, wherein
the content of the (c) polyalkenoic acid contained in the second agent in the total of 100 parts by mass of the (c) polyalkenoic acid contained in the second agent and the (d) water contained in the second agent is more than 52.5 parts by mass and less than 75 parts by mass.

5. The dental resin-reinforced glass ionomer cement composition for luting according to any one of claims 1 to 4, wherein
the acidic group in the (c) polyalkenoic acid is a carboxy group.

6. The dental resin-reinforced glass ionomer cement composition for luting according to any one of claims 1 to 5, wherein
the dental resin-reinforced glass ionomer cement composition for luting furher contains (f) acidic component having a molecular weight of 500 or less.

7. The dental resin-reinforced glass ionomer cement composition for luting according to claim 6, wherein
the acidic group in the (f) acidic component having a molecular weight of 500 or less is a carboxy group.

8. The dental resin-reinforced glass ionomer cement composition for luting according to claim 7, wherein
the (f) acidic component having a molecular weight of 500 or less has two or more carboxy groups.

9. The dental resin-reinforced glass ionomer cement composition for luting according to any one of claims 6 to 8, wherein
the (f) acidic component having a molecular weight of 500 or less does not have a polymerizable group.
